# EUROPEAN PATENT SPECIFICATION

(11) **EP 3 781 050 B1**
(45) Date of publication and mention of the grant of the patent: **20.11.2024**
(21) Application number: 18915415.6
(22) Date of filing: 24.09.2018
(51) Int. Cl.: A61B 17/3205, A61B 17/00

(54) **FRUSTOCONICAL HAIR PUNCH**
KEGELSTUMPFFÖRMIGE HAARSTANZE
POINÇON DE CHEVEU TRONCONIQUE

(30) Priority: 17.04.2018 US 201862658720 P
(43) Date of publication of application: 24.02.2021
(73) Proprietor: Umar, Sanusi, Manhattan Beach, CA 90266 (US)
(72) Inventor: Umar, Sanusi, Manhattan Beach, CA 90266 (US)
(74) Representative: Dickerson, David
(86) International application number: PCT/US2018/052455
(87) International publication number: WO 2019/203882

(56) References cited:
- WO-A1-2018/009232
- WO-A1-2018/009232
- AU-B1- 2016 266 109
- US-A- 5 922 000
- US-A1- 2010 125 287
- US-A1- 2011 160 746
- US-A1- 2011 160 746
- US-A1- 2014 031 839
- US-A1- 2016 235 441
- US-A1- 2016 249 948
- US-B2- 8 876 847

## Description

### BACKGROUND

This invention relates to surgical instruments and, more particularly, to a punch for extracting hair follicles from the skin.

Hair transplantation is a surgical technique that involves moving skin containing hair follicles from one part of the body (the donor site) to bald or balding parts (the recipient site). Hair naturally grows in follicles that contain groupings of 1 to 4 hairs, and transplant techniques typically move the 1-4 hair "follicular units" from the donor site to the recipient site. The follicles of hair are typically removed from the donor site using punches of between 0.7 mm and 1.90mm in diameter. The punches are tubular bodies having a skin- contacting cutting end, and are typically mounted in a tool that causes the punch to rotate or oscillate as the punch is brought into contact with the donor site, but are sometimes used manually and sometimes adopted for use in robotic hair follicle excisions. Hair follicles are very easily broken (or transected) during the removal site, and broken follicles are unlikely to be successfully transplanted. Embodiments of the disclosed invention solve this problem.

Current punches used for in vivo excision of hair follicles (A process called follicular unit extraction - FUE) have several problems. First, graft damage from transection can reduce graft survival. Additionally, less bulky grafts and graft de-sheathing reduce graft survival. A heat injury from a fast-rotating punch rubbing against a resisting internal punch luminal wall can compromise graft survival. There is a need for secondary dissection because of retained anchoring system, which makes the procedure more tedious and opens the grafts up to stress injuries from rough handling. At the same time, a problem of depth control as the punch gives no indication when it has been driven in too deep which leads to be transected and / or for the grafts disappearing into the punch and needing to be retrieved. Another problem is that of "buried grafts" which happens when the punch cutting end instead of cutting around and separating the grafts from its attachments and freeing it; it instead pushes the follicle irretrievably deep into the skin where it gets lodged, lost to the operator and potentially forms cysts and abscess. Further information pertaining to the prior art can be found in US 2011/160746 that discloses a punch for removing hair follicles from a donor site comprises a generally tubular body having a proximal end region terminating in a tissue-cutting edge, a distally-extending segment of reduced outer diameter-preferably a generally concave segment-terminating at its proximal end at or near the cutting edge and terminating at its distal end with a diameter substantially the same or greater than the diameter of the cutting edge. The punch further includes a follicle-receiving chamber defined by an inner wall extending distally from the proximal end of the punch. Preferably, at least a portion of the chamber lying within the external region of reduced diameter is generally converging in the distal direction.

WO 2018/009232 discloses a system, method, and apparatus for harvesting follicular units from an epidermis. The disclosed apparatus includes a hollow tubular structure having a central axis and a trumpet bell structure that is attached to an end of the hollow tubular structure. The trumpet bell structure terminates at a flat annular face that is substantially in a plane perpendicular to the central axis. The flat annular face has a sharp outer edge, and an inner surface of the trumpet bell structure inward from the flat annular face is smoothly varying.

### SUMMARY

The present invention provides a punch in accordance with independent claim 1. Preferred embodiments of the invention are reflected in the dependent claims.

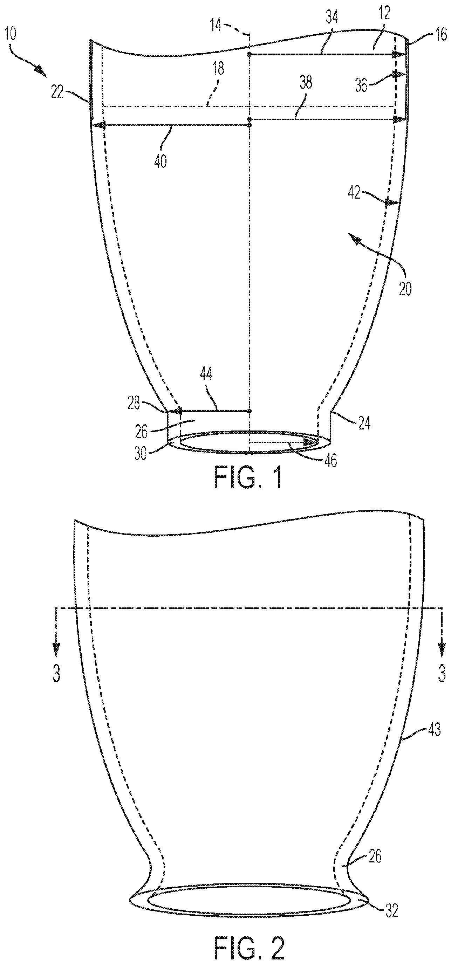

A punch can be used for removing hair follicles. The punch according to present disclosure comprises a generally tubular body formed about a generally central axis and extending from a generally tubular body proximal end to a generally tubular body distal cutting end. A generally frustoconical portion (as shown in Figures 1-42) and terminating in a distal cutting end, connected to the generally frustoconical proximal portion with or without a straight walled cylindrical transition zone of variable length ranging from 0-3mm. The cutting ends outer perimeter is defined by a sharp cutting tip which extends and points away from the central axis in angles ranging from 30 degrees to 95 degrees to direct the cutting forces substantially away from hair follicle. The cutting end creates a wound to remove hair follicles. The frustoconical gradient represents an angular expansion in the generally frustoconical portion between the proximal end and the distal end of the frustoconical portion generally defined by a length of at least one but no more than six millimeters.

In some embodiments, the external wall of the cutting end relative to the punch's general central axis on a lateral profile is straight. The external wall of the cutting end relative to the punch's general central axis on a lateral profile can be curved in a concave manner to terminate in a cutting tip that extends and points outward. While a substantially smooth concave segment is preferred, the segment can alternatively be stepped or angled without departing from the scope of the invention, and these variations are intended to be included within the term "concave" as used herein.

In some embodiments, a second frustoconical end (shown in Figure 1, component 24) can continue in a circular concave course that terminates at a cutting edge that points outwards and downwards relative to the central axis. The second frustoconical end can continue in circular concave course that terminates at a cutting edge that points outwards and at 90 degrees relative to the central axis. The second frustoconical end can continue in a circular concave course that terminates at a cutting edge that points outwards and proximally at between 95-120 degrees relative to the central axis.

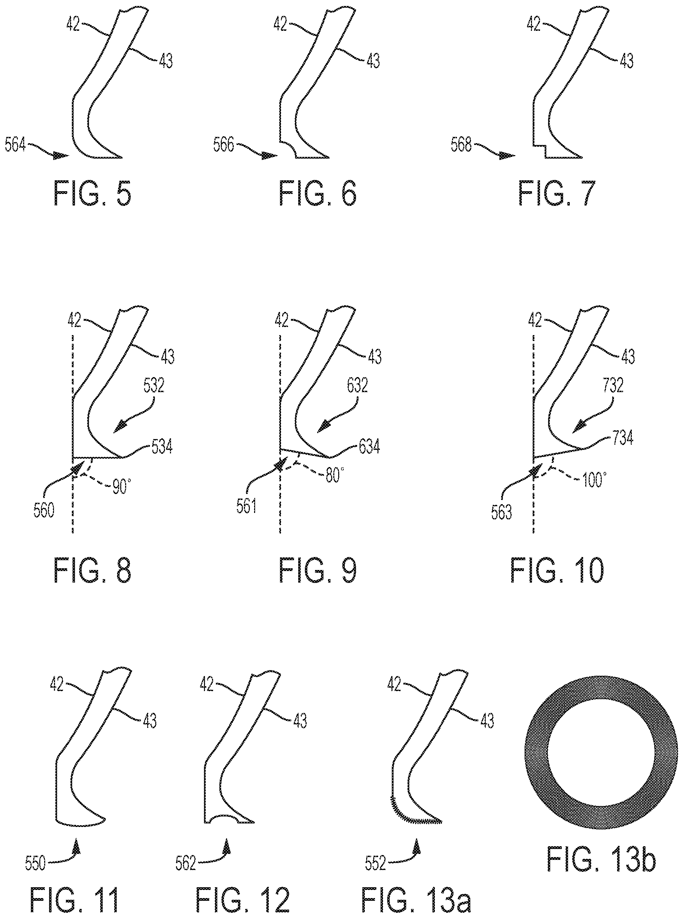

The cutting end relative to the punch's general central axis on a lateral profile that is viewed from the punch center to the cutting tip that can terminate in a flat plane at 90 degrees (as shown in FIG 8). The cutting end relative to the punch's general central axis on a lateral profile that is viewed from the punch center to the cutting tip that can be slanted in an angle that ranges from 85 degrees to 20 degrees in a manner that increases the sharpness of the cutting end with decreasing angulation (as shown in FIG 9). The cutting end relative to the punch's general central axis on a lateral profile that is viewed from the punch center to the cutting tip can terminate in an elevated flat plane at between 95 degrees and 145 degrees (as shown in FIG 10). The cutting end relative to the punch's general central axis on a lateral profile can terminate in an outwardly pointing sharp cutting edge. The cutting end surface viewed from its distal inferior face viewed from the punch's center to the outer margins where the cutting tip is located, can be concave (as shown in FIG 12). The cutting end surface viewed from its distal inferior face viewed from the punch's center to the outer margins where the cutting tip is located, can be convex (as shown in FIG 11).

The interface between where the largely vertical or proximally diverging internal wall of the frustoconical chamber and where it begins to diverge distally towards the laterally located cutting edge located on the external wall can be a smooth concave surface (as shown in Figure 6). The interface between where the largely vertical or proximally diverging internal wall of the frustoconical chamber and where it begins to diverge distally towards the laterally located cutting edge located on the external wall can be a smooth convex surface (as shown in Figure 5). The interface between where the largely vertical or proximally diverging internal wall of the frustoconical chamber and where it begins to diverge distally towards the laterally located cutting edge located on the external wall can be a ridged wall. The interface between where the largely vertical or proximally diverging internal wall of the frustoconical chamber and where it begins to diverge distally towards the laterally located cutting edge located on the external wall can be angled or stepped (as shown in Figure 7).

The cutting end surface viewed from its distal inferior face viewed from the punch's center to the outer margins where the cutting tip is located can be smooth. The cutting end surface viewed from its distal inferior face viewed from the punch's center to the outer margins where the cutting tip is located can be textured (as shown in Figure 13a) to aid in pulling grafts upwards into the punch lumen to prevent incidents of buried grafts associated with blunt and flat punches. The texturing can extend to the cutting tip or close to it. The texturing can assist in driving the punch forward to overcome the resistance to the punch distal progression into the tissue posed by the furstoconical gradient. The texturing can include lines of discontinuities approximately 12.5 micrometers deep. The texturing can include lines of discontinuities are generally concentric about the generally central axis. The texturing can include generally lines of discontinuities that are approximately 0.15 mm apart. The textured region can extend approximately 360° about the punch. The textured region can comprise generally helical lines of discontinuities in the external surface of the punch.

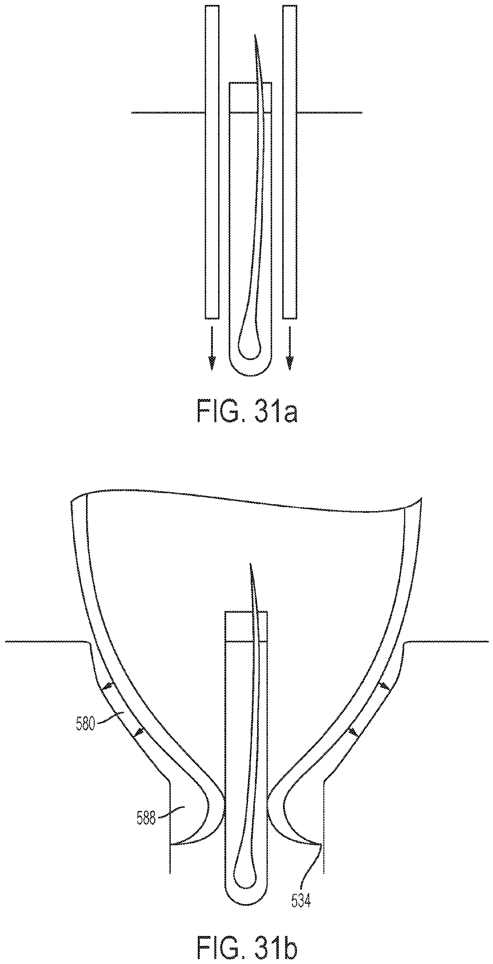

The frustoconical gradient can allow penetration of skin that is proximate to the hair follicle while presenting a resistance, while advancing, with an innate depth control mechanism so that the punch does not progress beyond a length of the punch (as shown in Figure 31b). The frustoconical gradient can allow penetration of skin that is proximate to the hair follicle while presenting a resistance, while advancing, causes a resistance to build up against the rotating punch causing it in a setting of low torque to slow down to a stop or near stop in the process impeding the cutting action of the punch to add to its ability to assert an innate depth control mechanism so that the punch does not progress beyond a length of the punch. The control mechanism can be communicatively coupled to the motor driven driveshaft and programmed with instructions to retard or stop the driveshaft speed of the motor-driven driveshaft as the punch penetrates the skin deeper to engage the frustoconical section. The frustoconical gradient can allow penetration of skin that is proximate to the hair follicle while presenting a resistance, while advancing, causes a resistance to build up against the rotating punch causing it in the setting of low torque to stop followed by a brief oscillatory or seesaw movement.

In some embodiments, the generally frustoconical portion can further comprise a generally frustoconical portion proximal end radius measured from the generally central axis to a generally frustoconical portion inner wall at the generally frustoconical portion proximal end. A generally frustoconical portion distal end radius can be measured from the generally central axis to a generally frustoconical portion inner wall at the generally frustoconical portion distal end. A ratio of the generally frustoconical portion proximal end radius to the frustoconical portion distal end radius is between 4:1 and 1.12 over a transition zone of between 1 mm and 7 mm.

In some embodiments, the generally tubular body can further comprise a generally tubular body proximal end radius measured from the generally central axis to a generally tubular body inner wall at the generally tubular body proximal end. A generally tubular body distal end radius can be measured from the generally central axis to a generally tubular body inner wall at the generally tubular body distal end. The generally tubular body proximal end radius can be approximately equal to the generally tubular body distal end radius.

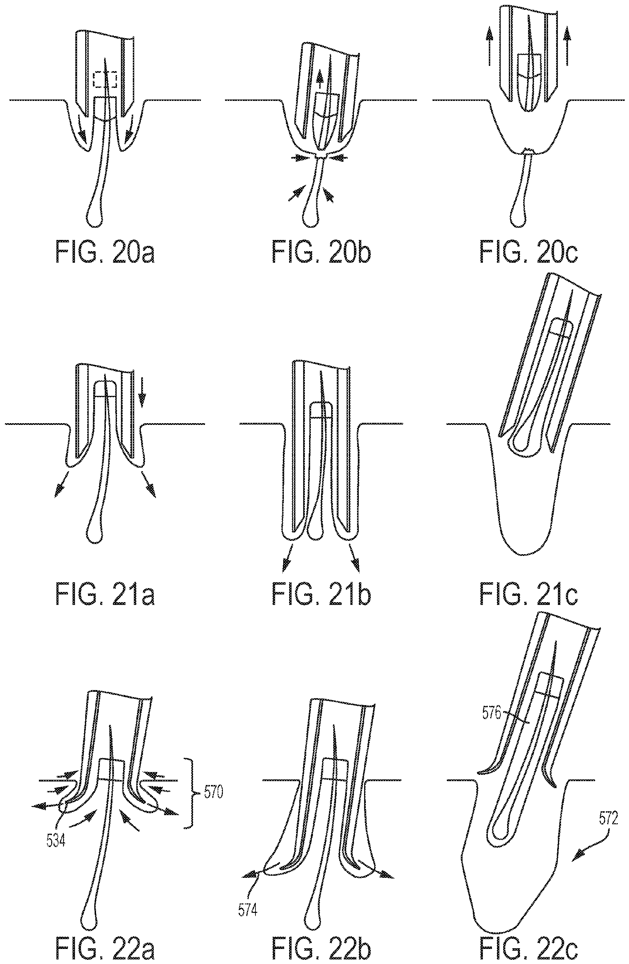

In some embodiments, the cutting edge can be configured to reduce wound edge evertion (as shown in Figure 20-25). The cutting edge can be configured to gather more tissue around the follicles as it scores around it creating bulkier grafts.

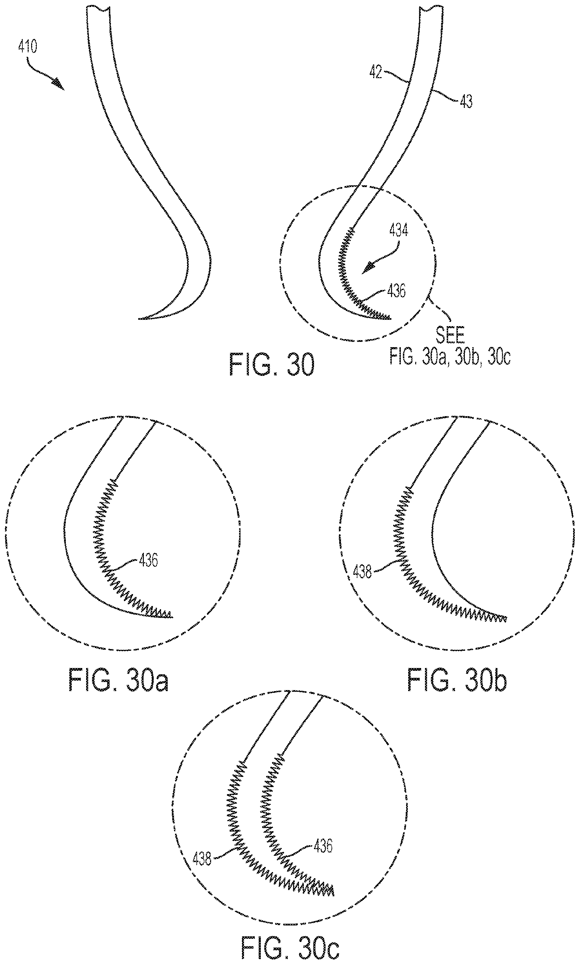

In some embodiments, a distal end region of the punch can have an exterior textured region that does not extend through a thickness of the generally cylindrical body (as shown in Figure 30a). The textured region can extend from the cutting tip proximally to a distance that ranges from 0.5 to 5mm. The texturing can help to generate a corkscrew effect which drives the punch forward to help overcome resistance to forward movement of the punch caused by the expanding frustoconical gradient. The punch can rotate 10-60 degrees relative to the skin surface and the punch tip engaging the skin with an initial cut, the texturing upon engaging the skin helps guides the punch tip forward in a screw like manner towards the follicle using the hair shaft as guide to safely score around the follicle while minimizing graft damage. The exterior textured region can be composed of lines of discontinuities approximately 12.5 micrometers deep. The lines of discontinuities can be generally concentric about the generally central axis. The exterior textured region can include generally lines of discontinuities that are approximately 0.15 mm apart. The exterior textured region extends approximately 360° about the punch. The exterior textured region can be formed by discontinuous areas of surface discontinuity. The exterior textured region can comprise generally helical lines of discontinuities in the external surface of the punch.

The distal end region of the punch can have an internal textured region that does not extend through the thickness of the generally cylindrical body (as shown in Figure 30b). The internal textured region can comprise lines of discontinuities in an internal surface of the follicle-receiving chamber. The lines can extend substantially 360° around the chamber. The lines can be approximately 12.5 micrometers deep. The lines can be approximately 0.15 mm apart. The generally curved concave exterior segment can have proximal and distal ends of substantially equal outer diameter. The textured region can extend from the cutting tip to a distance that ranges from 0.5 to 5mm proximally from the cutting tip. The textured region can extend proximate the cutting tip to a distance that ranges from 0.5 to 5mm proximally from the cutting tip.

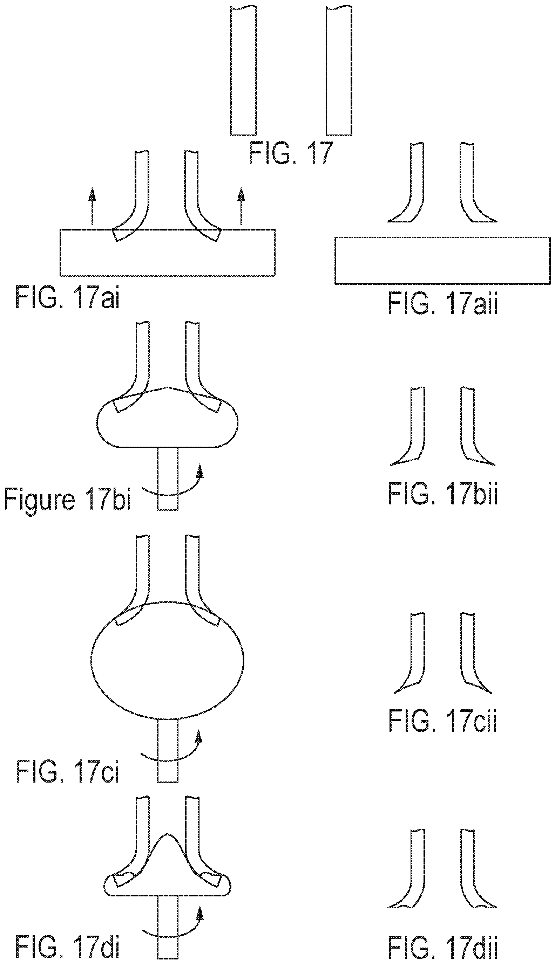

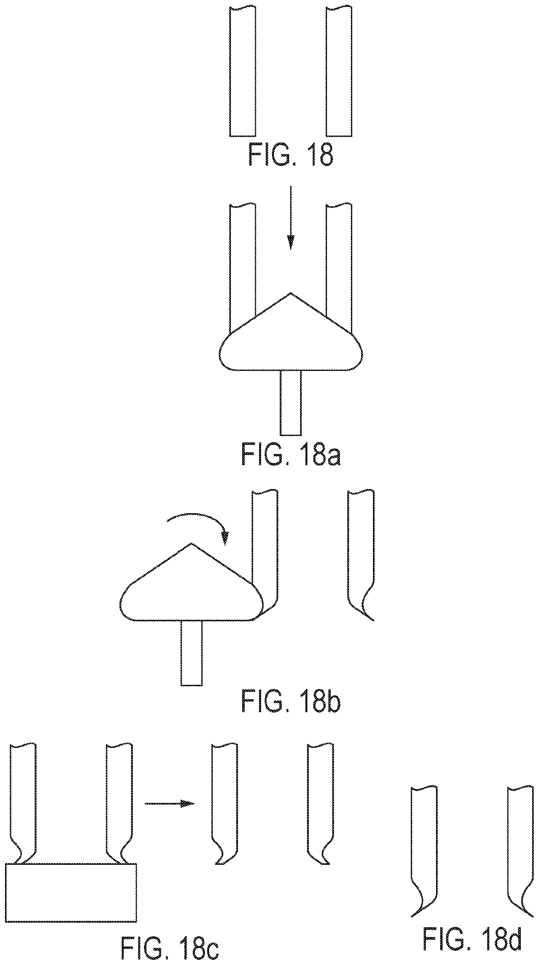

In some embodiments, the outwardly pointing cutting tip can be created by sharpening or beveling the tip from the proximal surface (as shown in Figure 18b). The outwardly pointing cutting tip can be created by honing or beveling the tip from the distal or inferior surface using a grinding stone or similar instrument (as shown in Figure 17ai). The outwardly pointing cutting tip can be created by sharpening or beveling the tip from the internal surface and external surface (as shown in Figure 17b, c and d). The outwardly pointing cutting tip can be created by sharpening or beveling the tip from the inferior surface and the external proximally facing surface (as shown in Figure 18). The outwardly pointing cutting tip can be created by deepening the concave grooving that circumferentially defines or enhances the concavity that extends distally to form the proximal surface of the tip. The outwardly pointing cutting tip can be created by sharpening or beveling the tip from the internal surface. The outwardly pointing cutting tip is a more rounded cutting tip where the angle where the two edges of the cutting tip meets comes to a more rounded blunter tip.

In some embodiments, the concavity can be formed by etching out a concave grove on the distal end of the external wall to form a concave groove that proximally form a second frustoconical portion that begins distal to the already described proximal frustoconical portion and terminating in the middle of the concave groove it defines. A second frustoconical end can continues in a circular concave course that terminates at a cutting edge that points outwards and downwards relative to the central axis. A second frustoconical end can continue in a circular concave course that terminates at a cutting edge that points outwards and at 90 degrees relative to the central axis (as shown in Figure 8). A second frustoconical end can continue in circular concave course that terminates at a cutting edge that points outwards and proximally at between 20-85 degrees relative to the central axis (as shown in Figure 9). A second frustoconical end can continue in circular concave course that terminates at a cutting edge that points outwards and proximally at between 95-120 degrees relative to the central axis (as shown in Figure 10).

A hand piece can be used for engaging a hair punch in order to remove a hair follicle from skin. The hand piece comprises a hand piece body extending along a generally central axis for holding a follicle punch having a cutting end. A motor-driven driveshaft is mounted within the handpiece body for a slightly offset rotation from the generally central axis for rotating the follicular punch when the follicular punch is held by the handpiece.

In some embodiments, a fluid path carried by the handpiece for conducting a liquid from a source external to the handpiece into the lumen of the follicle punch can be held by the handpiece for delivery of the fluid to the cutting edge of the follicle punch. The slightly offset rotation can be arranged by slightly rotating the follicle punch from the generally central axis by at least one degree and no more than 10 degrees. The slightly offset rotation can be arranged by mounting the motor-driven drive shaft away from the generally central axis by at least 1 mm but no more than 10 mm. The follicle punch can score around the follicle cutting tissue around the hair follicle in order to separate the hair follicle from the skin. In some embodiments, a motor, driving the motor-driven driveshaft wherein the motor can run at least 0.2 amps but no more than 0.8 amps. This range allows for significant slowing of punch when the shoulder of the punch presses on tissue and increases resistance with progressing depth of penetration.

A process for removing a hair follicle from skin includes the following steps. First, contacting the skin with a follicle punch making an initial score. A handpiece body extends along a generally central axis for holding a follicle punch having a cutting end the follicle punch further comprises a motor-driven driveshaft mounted within the handpiece body. Then, inserting the cutting edge into the skin around a hair follicle perimeter. An increasing friction force caused by the skin on the follicle punch is approximately equal to a limit switch torque on a motor driving the motor-driven driveshaft. The follicle punch further comprises a generally tubular body formed about a generally central axis and extending from a generally tubular body proximal end to a generally tubular body distal cutting end. A generally frustoconical portion and terminating in a distal cutting end, connected to the generally frustoconical proximal portion with or without a straight walled cylindrical transition zone of variable length ranging from 0-3mm. The cutting ends outer perimeter is defined by a sharp cutting tip which extends and points away from the central axis in angles ranging from 30 degrees to 95 degrees to direct the cutting forces substantially away from hair follicle. The cutting end creates a wound to remove hair follicles. The frustoconical gradient represents an angular expansion in the generally frustoconical portion between the proximal end and the distal end of the frustoconical portion generally defined by a length of at least one but no more than six millimeters. The friction increases substantially with depth due to the expanding external wall diameter of the frustoconical punch (as shown in Figure 31b) to result in slowing or stoppage of a rotation of the punch causing halting or slowing further punch progression in an innate depth control. The friction increases substantially with depth due to the expanding external wall diameter of the frustoconical punch to result in slowing or stoppage of the punch rotation halting or slowing further punch progression to minimize chances of cutting into the follicle. The frustoconical gradient allows penetration of skin that is proximate to the hair follicle while presenting a resistance while advancing that causes a resistance to build up against the rotating punch causing it in the setting of low torque to stop or near stop which minimizes the cutting action in deeper scores in the process reducing the chance of the punch cutting into and damaging the graft.

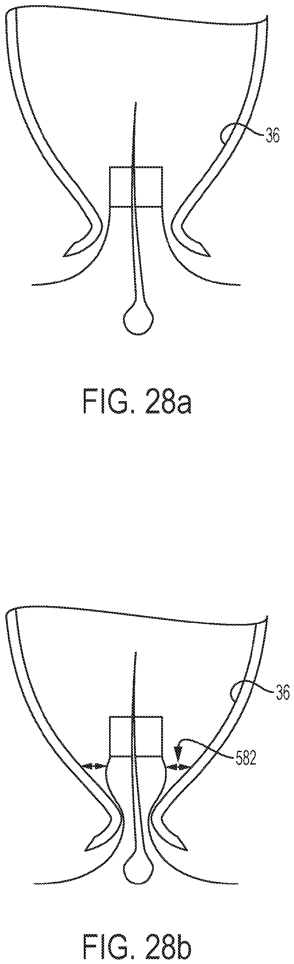

In some embodiments, the frustoconical gradient and the volume expansion inherent to it in a rapidly rotating punch can generate a weak suction force that assists with graft pulling action. The frustoconical gradient can create a capacious chamber in skin proximate the hair follicle in order to accommodate a large hair follicle and minimize torsion injury that results from frictional forces that occur when the hair follicle is impacted (as shown in Figure 28b).

A process for removing a hair follicle from skin incvolves contacting the skin with an internal wall textured follicle punch making an initial score. A handpiece body extends along a generally central axis for holding a follicle punch having a cutting end the follicle punch further comprises a motor-driven driveshaft mounted within the handpiece body. The distal end region of the punch's internal wall has textures or grooves that do not extend through the thickness of the generally cylindrical body which comprising of part concentric, partly helical, lines of discontinuities along its 360 degrees zone, each approximately 2.5 micrometers in depth, spread approximately 0.15mm apart to create a mini suction vortex and or a corkscrew effect that pulls up the graft into the punch lumen. The effect of the internal texturing enables that when the rapidly rotating punch is tilted at a 1 degree to 90 degree angle to the skin at the hair follicle exit point with the hair shaft projecting into its lumen, the texturing results in a screw like effect that drives and guides the punch's distal cutting end to navigate around the follicle through its deeper path through the skin compensation for punch-follicle misalignments as well as hair angulations in the process with lesser user control.

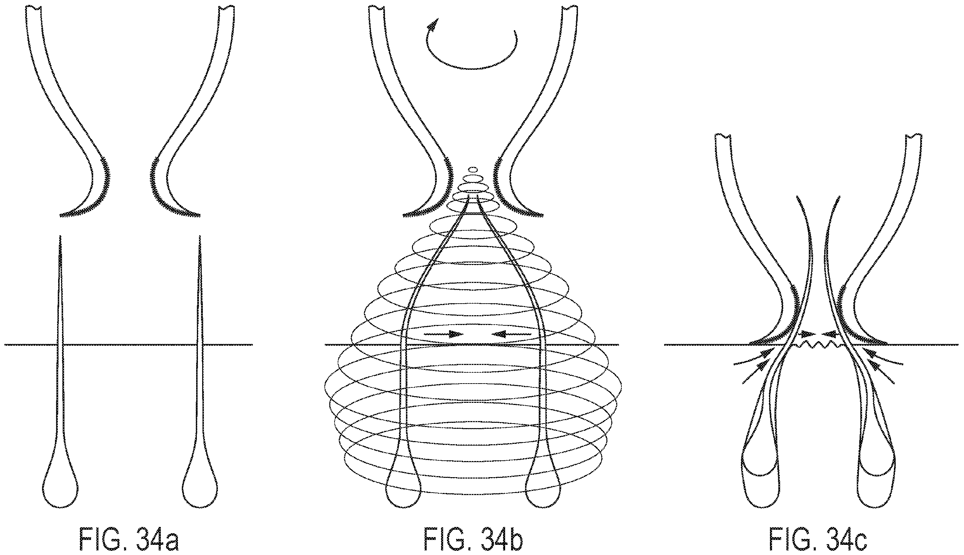

In some embodiments, the internal texturing in a rapidly rotating punch can generates a vortex effect that begins as the punch approaches the skin result in gathering of hair shafts of even widely dispersed follicular unit to enable the punch to gather in grafts whose diameter of skin spread exceeds the maximum diameter of the cutting end of the punch (as shown in Figure 34). The process provides for minimization of the incident of buried grafts in the face of blunt or flat tipped punches. Operator skill can be minimized in exactly aligning grafts with the hair follicle to minimize transection.

A process for removing a hair follicle from skin can include contacting the skin with a textured follicle punch making an initial score. A handpiece body extends along a generally central axis for holding a follicle punch having a cutting end the follicle punch further comprises a motor-driven driveshaft mounted within the handpiece body. The textured follicle punch comprises a generally tubular body formed about a generally central axis and extending from a generally tubular body proximal end to a generally tubular body distal cutting end. A generally frustoconical portion terminates in a distal cutting end, connected to the generally frustoconical proximal portion with or without a straight walled cylindrical transition zone of variable length ranging from 0-3mm. The cutting ends outer perimeter is defined by a sharp cutting tip which extends and points away from the central axis in angles ranging from 30 degrees to 95 degrees to direct the cutting forces substantially away from hair follicle. The cutting end creates a wound to remove hair follicles. The effect of the expanding volume caused by the frustoconicallity enables that in a rapidly rotating punch a suction energy or vortex is generate a Bernoulli Effect which pulls up the graft when the rim of the punch cups and forms a tight seal around the perimeter of the follicle. The graft pulling effect helps to guide the follicle up the punch lumen ahead of the cutting end to assist in correcting for punch follicle misalignment and hair angulation deep to the skin.

In some embodiments, the process can further comprise screwing the follicle punch into the skin at a rotational speed of less than one revolution per second. The process can further comprise arranging longitudinal groves on an outer surface of the follicle punch; wherein the longitudinal grooves that guide penetration when the follicle punch is used in a plunging manner rather than a rotary manner. Then, horizontal groves on an outer surface of a frustoconical portion of the follicle punch; wherein the horizontally disposed lines of discontinuities part concentric, part helical grooves that guides and drives penetration when the a rotary manner. After that, suctioning a fluid through a lumen in the follicle punch. Following that, increasing an inner diameter of an interior wall of the follicle punch in order to create a suction vortex due to a Bernoulli effect within the follicle punch. In some embodiments, the punch can be inserted into the skin with mechanical movement that is rotary,

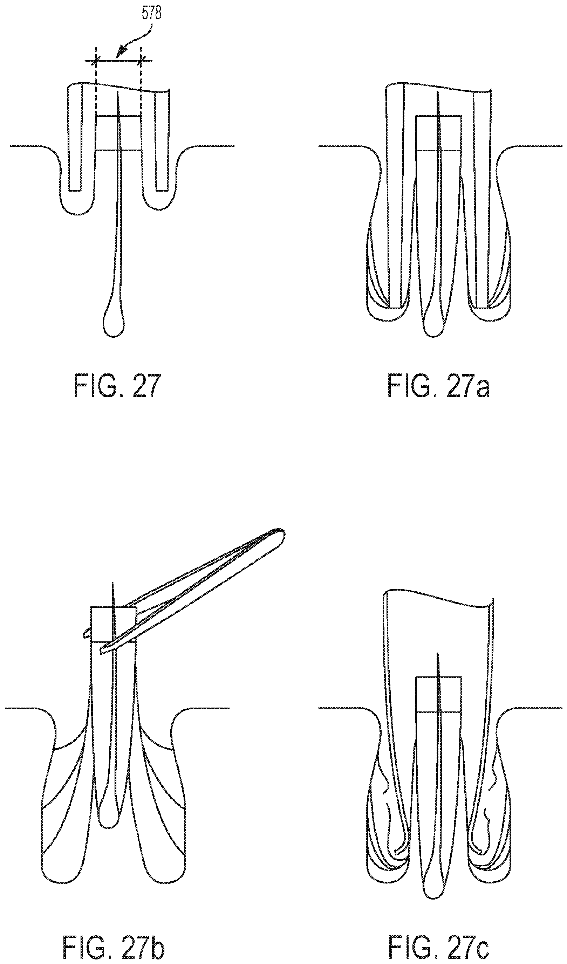

A process for removing a hair follicle from skin includes contacting the skin with a textured follicle punch making an initial score. A handpiece body extends along a generally central axis for holding a follicle punch having a cutting end the follicle punch further comprises a motor-driven driveshaft mounted within the handpiece body. The textured follicle punch comprises a generally tubular body formed about a generally central axis and extending from a generally tubular body proximal end to a generally tubular body distal cutting end. A generally frustoconical portion and terminating in a distal cutting end is connected to the generally frustoconical proximal portion with or without a straight walled cylindrical transition zone of variable length ranging from 0-3mm. The cutting ends outer perimeter is defined by a sharp cutting tip which extends and points away from the central axis in angles ranging from 30 degrees to 95 degrees to direct the cutting forces substantially away from hair follicle. The cutting end creates a wound to remove hair follicles. The effect of the expanding volume caused by the frustoconicallity enables that in a rapidly rotating or oscillating punch, as the punch tip is progressively inserted into the deeper portions of the skin with the follicle engulfed within its lumen, in horizontal cross section the expanding external diameter brought about by the frustoconicallity results in blunt dissection of the tissue apparatus that attaches the hair follicles connective tissue sheath to the surrounding skin layers (as shown in Figure 27c). The dissection process is blunt since the tissues are peeled away from the follicle by the expanding external wall diameter without the effect of a sharp cutting element instead relying on the smooth or favorably textured wall of the frustoconical external wall surface as it expand away from the follicle located in its lumen.

In some embodiments, the blunt dissection can minimize the need for secondary dissection to free the grafts following an initial score to result in increased number of grafts coming free with only the single scoring stroke of the punch. The blunt dissection can minimize the need for secondary dissection to the extent that a substantial number of the grafts can be retrieved by swiping the extraction zone with a frictional element such as a gauze.

A process for removing a hair follicle from skin, can include contacting the skin with a textured follicle punch making an initial score; wherein a handpiece body extends along a generally central axis for holding a follicle punch having a cutting end the follicle punch further comprises a motor-driven driveshaft mounted within the handpiece body.

The textured follicle punch further comprises a generally tubular body formed about a generally central axis and extending from a generally tubular body proximal end to a generally tubular body distal cutting end and a generally frustoconical portion and terminating in a distal cutting end, connected to the generally frustoconical proximal portion with or without a straight walled cylindrical transition zone of variable length ranging from 0-3mm. The cutting ends outer perimeter is defined by a sharp cutting tip which extends and points away from the central axis in angles ranging from 30 degrees to 95 degrees to direct the cutting forces substantially away from hair follicle. The cutting end creates a wound to remove hair follicles. The effect of the expanding volume enables that in the scoring process when the top portion of the follicle is scores and contained within the first 1-3 mm of the punch distal lumen with the distal end of the punch still tethered to surrounding tissue attachment, the twisting motion of the graft top that would occur when the follicle top adheres to the wall of a conventional punch is cancelled since the punch is significantly roomed to minimize an adhesion that is necessary to cause the twisting motion of the top portion of the graft. In some embodiments, the process futher comprieses minimizing torsion mediated graft damage in in vivo follicular unit excision procedures.

A process for removing a hair follicle from skin includes contacting the skin with a follicle punch making an initial score. The follicle punch has a handpiece body extends along a generally central axis for holding a follicle punch having a cutting end the follicle punch further comprises a motor-driven driveshaft mounted within the handpiece body. A fluid path is carried by the handpiece body for conducting a liquid from a source external to the handpiece into the lumen of the follicle punch held by the handpiece for delivery of the fluid to the cutting edge of the follicle punch.

The fluid can minimize heat build up in the interface between the rotating punch and the grafts. The fluid can serve as a lubricant minimizes friction between the graft and the punch tip and distal internal walls as its ascends up the punch lumen. The punch distal rim can engages the skin in the perimeter of the hair follicles it the fluid forms a tight water seal which serves to increase the graft pulling action of the rotating punch. Misalignments and deep skin hair angulation can be compensated for as a result. When the fluid irrigation system deposits a life sustaining physiologic fluid or a nutritious fluid or oxygen giving fluid into the scored hole to sustain the grafts after it has been substantially separated from its natural source of succor that derives from its attachment to its attachments.

In some embodiments, the fluid system can be attached to a pulse fluid ejection mode that upon activation releases a jet of fluid through the tip of the punch to affect the egress of any stuck graft or follicular debris making operation of the handpiece hands free. This can result in hands free retrieval of impacted grafts removing the need for stopping procedure to remove impacted grafts with forceps or sacrificing it by pushing it into the proximal portions of the punch in to enable continuation of the procedure.

A process for removing a hair follicle from skin can involve contacting the skin with the follicle punch when used in a hair transplant robotic system.

A process for making a follicle punch can involve arranging a generally tubular body formed about a generally central axis and extending from a generally tubular body proximal end to a generally tubular body distal end. Then, arranging a generally frustoconical portion, further comprising a generally frustoconical portion proximal end and a generally frustoconical portion distal end; formed about the generally central axis. The generally frustoconical portion distal end is joined to the generally tubular body proximal end. After that, arranging a cutting edge, connected to the generally frustoconical portion; wherein the cutting edge extends away from the central axis.

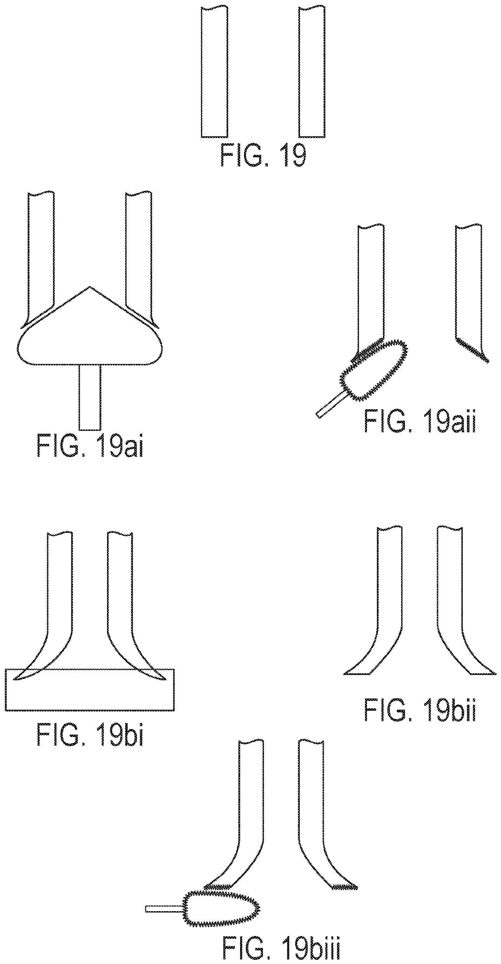

In some embodiments, the process can further involve texturing of the outer wall of the distal 1-3mm of the cutting end by etching using an appropriately grit and appropriately shaped grinder stone or metallic bits with either the grit or punch rotating against the other. Additionally, texturing of inferior face also distal face of the cutting end by etching using an appropriately grit and appropriately shaped grinder stone or metallic bits with either the grit and / or punch rotating against the other (as shown in Figure 19). Reducing friction caused by the generally tubular body can be accomplished by polishing, lubricating, manipulating electrostatic properties of, and controlling a temperature of the generally tubular body.

In some embodiments, the process can further comprise arranging at least one opening in the generally tubular body. The process can further comprise arranging a cobblestone etching pattern on the generally tubular body. The process can further comprise arranging at least one etching pattern on the generally tubular body.

In some embodiments, the process can further comprise: forming a concave lateral profile, forming a convex lateral profile, forming a straight lateral profile, forming a curved lateral profile, rows of vertical slits itched into the external wall of the distal 1-3 mm of the distal end of the punch.

In some embodiments, the process can further comprise: forming a flat surface on the bottom of the cutting end between the junction where the internal wall turns outward to join the outward facing cutting end outer margin and then creating a hollow concave groove in the middle of the flat that forms a complete circle around the cutting end flat undersurface (as shown in Figure 12). In some embodiments, the concave groove upon engaging the skin in a rotating punch can form a suction pad to secure the attachment of the punch to the skin for a better grip wherein this enhances the punch - graft engagement.

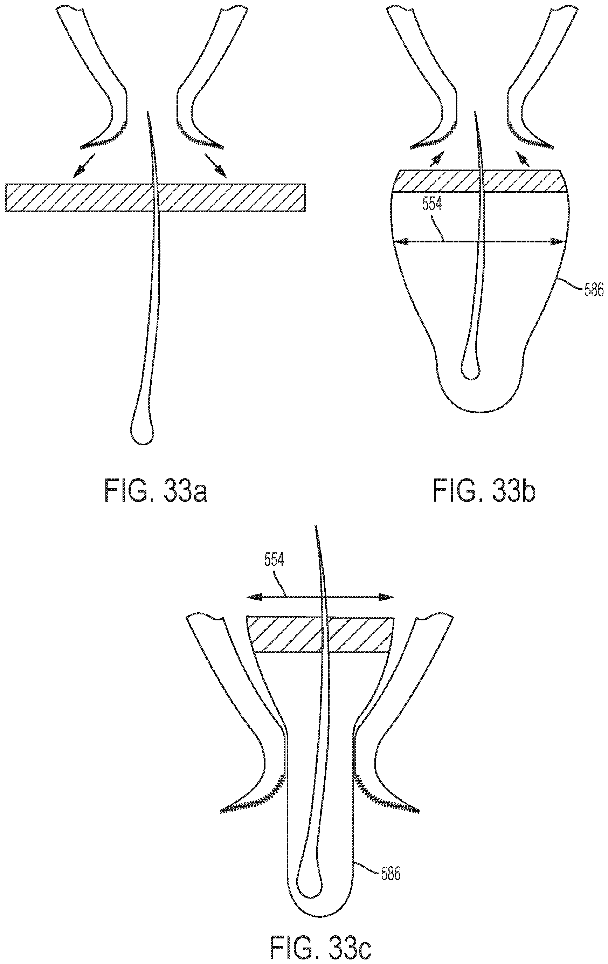

In some embodiments the process can be enhanced by the effect of fluid egressing mechanism which creates a fluid plane interface between punch cutting end and the skin. The surface of the cutting end viewed from the underside can be flat. The internal diameter of the section beginning from the end of the frustoconical portion extending distally to the remainder of the cutting end distally can be increased by etching of the internal wall a cylinder whose margin perimeter is created by removing the inner 25-75% of the tube wall thickness in the target zone. (as shown in Figure 30a-c) The flat blunt inferior surface can be slanted to diverge outwards and downwards to facilitate the sliding of grafts upwards and into the punch lumen to avoid graft burial. The flat blunt inferior surface can be textured by etching part concentric, partly helical, lines of discontinuities covering the cutting end inferior face along its 360 degrees zone, each approximately 2.5 micrometers in depth, spread approximately -0.15mm apart to create a mini suction vortex that generates a graft pulling effect in a rotating punch to the effect that pushes the grafts up into the punch lumen to avoid buried graft with concentric, slanted to diverge outwards and downwards to facilitate the sliding of grafts upwards and into the punch lumen to avoid graft burial (as shown in Figure 33a-c). The flat blunt inferior surface can be textured by etching part concentric, partly helical, lines of discontinuities covering the cutting end inferior face along its 360 degrees zone, each approximately 2.5micrometers in depth, spread approximately -0.15mm apart to create a mini suction vortex that generates a graft pulling effect in a rotating punch to the effect that pushes the grafts up into the punch lumen to avoid buried graft with concentric, slanted to diverge outwards and downwards to facilitate the sliding of grafts upwards and into the punch lumen to avoid graft burial(as shown in Figure 33a-c). The surface of the cutting end viewed from the underside can be convex.

In some embodiments, concavity of the external wall at the distal 1-4 mm used to create the flare in the cutting tip can be created by first flaring the distal end of the tube, followed by grinding out of the underside of the cutting end using a pyramidal shaped grinding stone or metal by rotating either/or both grinder or punch along a coalition course with an apex of the grinder dead center positioned into and parallel to the punch lumen center (as shown in Figure 17a-d).

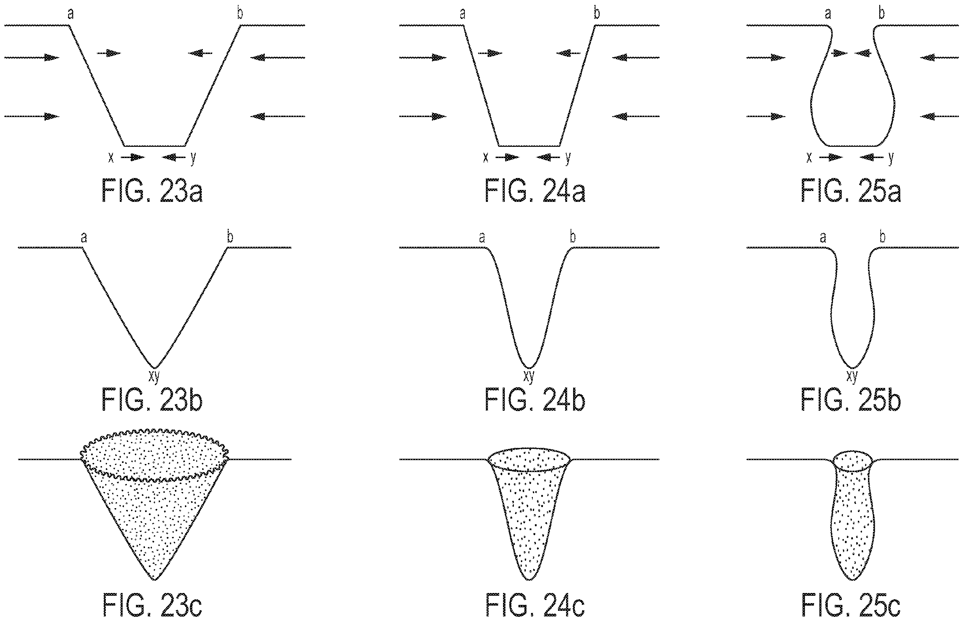

Wounds created by conventional FUE punches which have a cutting end which is beveled from the outside have a cutting axis that is directed towards the central axis of the punch tube. They consequently tend to create substantially everted wounds (V shaped configuration) as the punch is inserted into the skin (as shown in Figure 20A-C). This configuration of a wound upon contraction would close in the deeper portions leaving a relatively larger gap on the skin surface (as shown in Figure 23A-B). This would result in the larger gap closing by a process called second intention healing (SIH) with a plug of a relatively larger scar (as shown in Figure 23C). Conversely, Inner beveled punches whose cutting axis relative to the outer beveled punches is directed less centrally, would create relatively less everted wounds (as shown in Figure 21A-B) and minimize the size of the scar (as shown in Figure 24A-C). Punches whose cutting tip is flared to point away from the central axis would for the same line of reasoning created substantially less everted wounds (as shown in Figure 22A-C) which would favor even lesser skin surface gap of wounds at contraction. Consequently, these flared tipped punches result in relatively lesser gap ofwound at the skin surface at contraction and ultimately less visible scarring (as shown in Figure 25A-C).

Another advantage of the outwardly flared tipped punches is that the cutting axis is substantially directed away from the central axis and hence the follicle entering the punch lumen. This results in a lesser chance of the cutting tip cutting into the follicle and transecting it. Instead the follicle abuts a nonthreatening surface of the cutting end of the punch (as shown in Figure 22A-B).

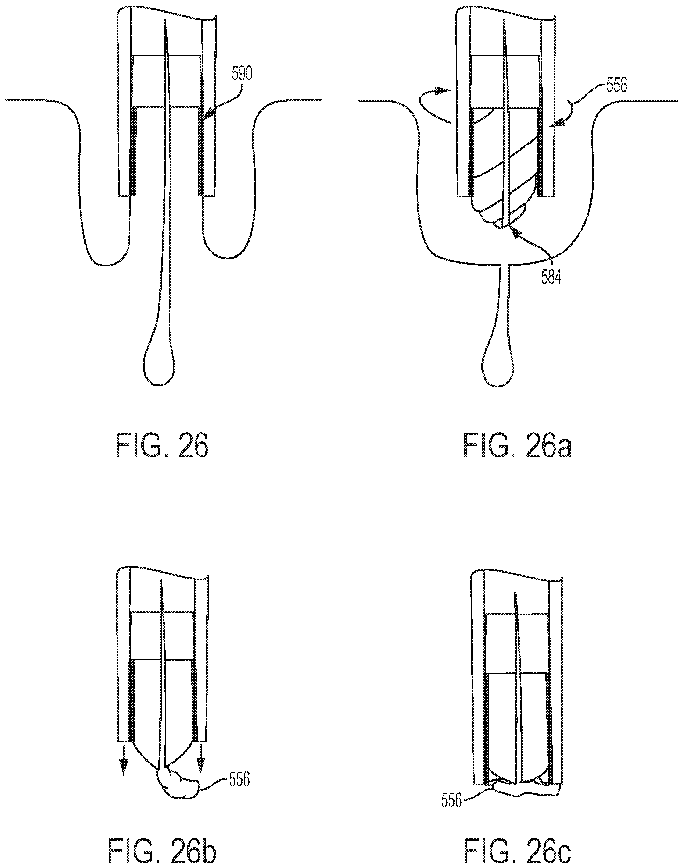

Graft damage can be caused by a number of factors including: Impaction causing torsion from grafts adhering to the luminal lining of the punch as it ascends and gather inside it. Once it is impacted, further rotation or oscillation of the punch results in transection (as shown in Figure 26A), so the punch needs to be stopped and cleaned. An impacted graft prevents further upward advancement up the punch lumen, further advancing of the punch results in compression of distal bulb end which is distorted, crushed (as shown in Figure 26B-C) and if it is a splayed follicle it becomes liable to transection upon further advancement of the punch. Depth control attempts to assuage this. Impacted grafts can be damage from forceps removal, neglected and inaccessible grafts. The need for secondary dissection is because of a retained anchoring system (as shown in Figure 27A-B), which makes the procedure more tedious and opens the grafts up to stress injuries from rough handling. Embodiments of the disclosed invention solve all this.

Hair follicles are easily damaged during the removal procedure at the donor site by frictional contact with the interior wall of the rotating punch, and by the punch's cutting end as it descends into the tissue. With respect to the frictional damage, there can come a point during the removal at which the follicle becomes impacted against the inner wall of a conventional punch to such an extent that the follicle rotates with the punch as essentially a single unit as the graft is gathered into the chamber. In the meantime, the opposite end of the follicle remains relatively substantially stationary, resulting in a twisting of the follicle until said opposite end is sheared off (as shown in Figure 26A). This is called "transection". The two transected parts of the follicle are less viable, and unsuitable for transplantation.

The shape of the chamber within a punch constructed in accordance with the invention, however, tends to permit the punch to rotate smoothly around the follicle. The shape of the chamber distal to the cutting edge of the punch increases the capacity of the punch lumen, thus reducing the friction between follicle and the punch's inner wall. Consequently, there is less temperature build-up, less damage to the follicular tissue, less incidence of impaction and, consequently, a reduction in the transection rate (as shown in Figure 28A-B).

Disclosed is a punch used to remove hair follicles. The punch includes a generally tubular body formed about a generally central axis and extending from a generally tubular body proximal end to a generally tubular body distal end. A generally frustoconical portion further includes a generally frustoconical portion proximal portion that terminates in a cutting end. Where in the cutting end is separated from the terminal end of the frustoconical portion by a cylindrical section with walls running parallel to the tube central axis in lengths that vary from 0mm to 3mm (as shown in Figure 1)

The generally tubular body can further comprise a generally tubular body proximal end radius measured from the generally central axis to a generally tubular body inner wall at the generally tubular body proximal end. A generally tubular body distal end radius is measured from the generally central axis to a generally tubular body inner wall at the generally tubular body distal end. The generally tubular body proximal end radius is approximately equal to the generally tubular body distal end radius.

The generally frustoconical portion can further comprise a generally frustoconical portion proximal end radius measured from the generally central axis to a generally frustoconical portion inner wall at the generally frustoconical portion proximal end. A generally frustoconical portion distal end radius is measured from the generally central axis to a generally frustoconical portion inner wall at the generally frustoconical portion distal end. The generally frustoconical portion distal end radius and the generally frustoconical portion proximal end radius can vary in a number of proportions.

### BRIEF DESCRIPTION OF THE FIGURES

The detailed description of some embodiments of the invention is made below with reference to the accompanying figures, wherein like numerals represent corresponding parts of the figures.
**FIG. 1** exemplarily illustrates a profile view of the frustoconical hair punch
**FIG. 2** exemplarily illustrates a profile view of the frustoconical hair punch
**FIG. 3** exemplarily illustrates the cross-sectional view of the frustoconical hair punch as shown in the parting line in FIG. 2.
**FIG. 4** exemplarily illustrates an embodiment of the frustoconical hair punch.
**FIG. 5** exemplarily illustrates embodiments of the cutting end region of the frustoconical hair punch.
**FIG. 6** exemplarily illustrates embodiments of the cutting end region of the frustoconical hair punch.
**FIG. 7** exemplarily illustrates embodiments of the cutting end region of the frustoconical hair punch.
**FIG. 8** exemplarily illustrates embodiments of the cutting end region of the frustoconical hair punch.
**FIG. 9** exemplarily illustrates embodiments of the cutting end region of the frustoconical hair punch.
**FIG. 10** exemplarily illustrates embodiments of the cutting end region of the frustoconical hair punch.
**FIG. 11** exemplarily illustrates embodiments of the cutting end region of the frustoconical hair punch.
**FIG. 12** exemplarily illustrates embodiments of the cutting end region of the frustoconical hair punch.
**FIG. 13** **a** exemplarily illustrates embodiments of the cutting end region of the frustoconical hair punch with an internally textured portion.
**FIG. 13 b** exemplarily illustrates an embodiment of the cutting end region with an internally textured portion of Figure 13A as seen from beneath looking upward.

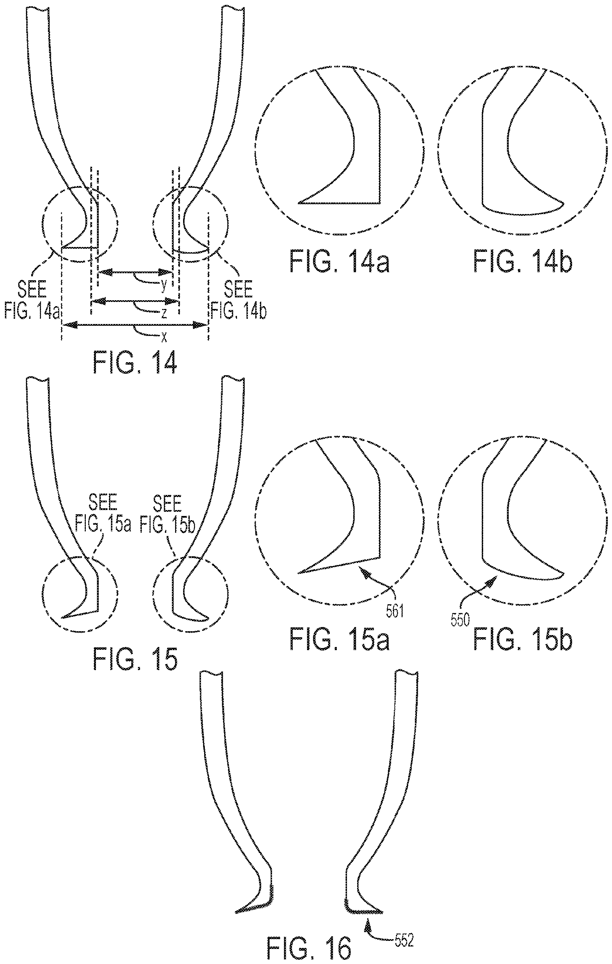

**FIG. 14** exemplarily illustrates embodiments of the cutting end region of the frustoconical hair punch.
**FIG. 14** a-b exemplarily illustrates embodiments of the cutting end region of the frustoconical hair punch.
**FIG. 15** exemplarily illustrates embodiments of the cutting end region of the frustoconical hair punch.
**FIG. 15 a-b** exemplarily illustrates embodiments of the cutting end region of the frustoconical hair punch.
**FIG. 16** exemplarily illustrates embodiments of the cutting end region of the frustoconical hair punch.
**FIG. 17** exemplarily illustrates sharpening or beveling embodiments of the frustoconical hair punch.
**FIG. 17** **ai-dii** exemplarily illustrates sharpening or beveling embodiments of the frustoconical hair punch.
**FIG. 18** exemplarily illustrates sharpening or beveling embodiments of the frustoconical hair punch.
**FIG. 18** a-d exemplarily illustrates sharpening or beveling embodiments of the frustoconical hair punch.
**FIG. 19** exemplarily illustrates means for texturing embodiments of the frustoconical hair punch.
**FIG. 19** **ai-biii** exemplarily illustrates means for texturing embodiments of the frustoconical hair punch.
**FIG. 20 a-c** exemplarily illustrates the use of the frustoconical hair punch.
**FIG. 21 a-c** exemplarily illustrates the use of the frustoconical hair punch.
**FIG. 22 a-c** exemplarily illustrates the use of the frustoconical hair punch.
**FIG. 23 a-c** exemplarily illustrates resultant effects of the frustoconical hair punch.
**FIG. 24 a-c** exemplarily illustrates resultant effects of the frustoconical hair punch
**FIG. 25 a-c** exemplarily illustrates resultant effects of the frustoconical hair punch.
**FIG. 26** exemplarily illustrates the use of the frustoconical hair punch.
**FIG. 26 a-c** exemplarily illustrates the use of the frustoconical hair punch.
**FIG. 27** exemplarily illustrates the use of the frustoconical hair punch.
**FIG. 27 a-c** exemplarily illustrates the use of the frustoconical hair punch.
**FIG. 28 a-b** exemplarily illustrates the use of the frustoconical hair punch.

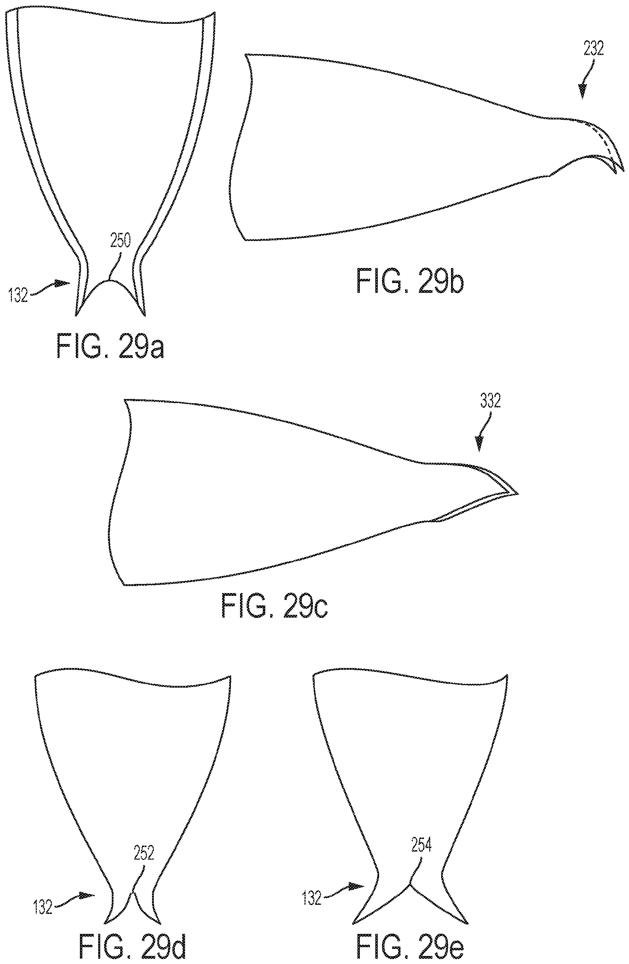

**FIG. 29 a-e** exemplarily illustrates a pronged for non-rotary embodiment for the frustoconical hair punch.
**FIG. 30** exemplarily illustrates embodiments of the cutting end region of the frustoconical hair punch.
**FIG. 30 a-c** exemplarily illustrates embodiments of the cutting end region of the frustoconical hair punch.
**FIG. 31 a-b** exemplarily illustrates the use of the frustoconical hair punch.

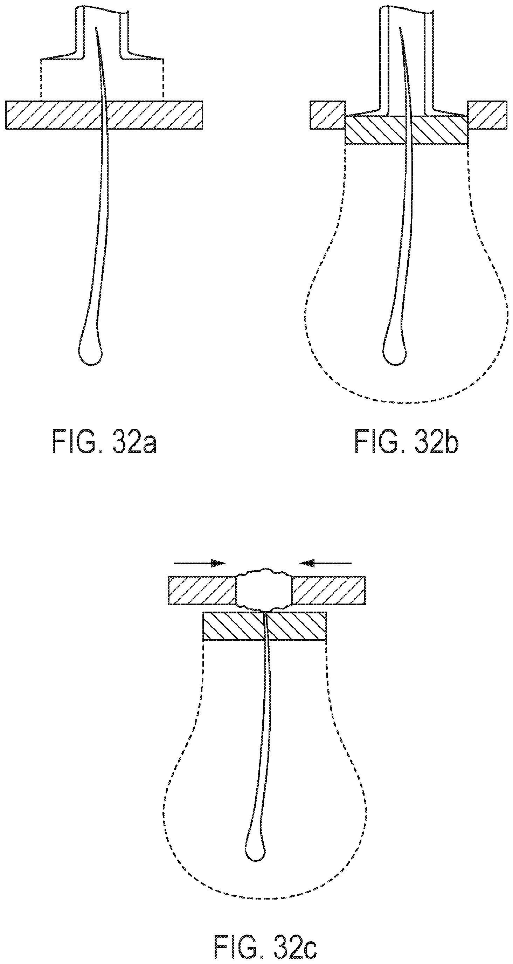

**FIG. 32 a-c** exemplarily illustrates resultant effects of the frustoconical hair punch.
**FIG. 33 a-c** exemplarily illustrates resultant effects of the frustoconical hair punch.
**FIG. 34 a-c** exemplarily illustrates the use of the frustoconical hair punch.

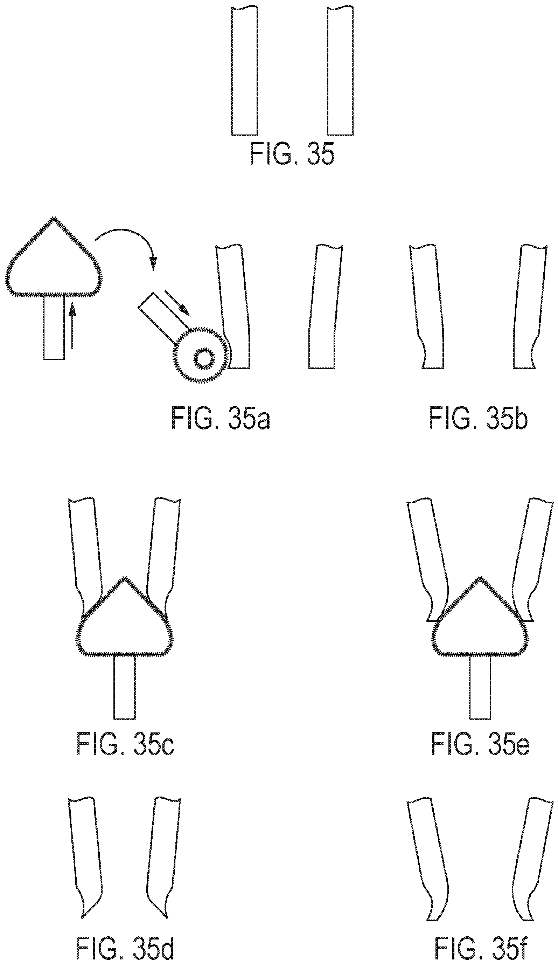

FIG. 35 exemplarily illustrates means for texturing embodiments of the frustoconical hair punch.
**FIG. 35 a-f** exemplarily illustrates means for texturing embodiments of the frustoconical hair punch.

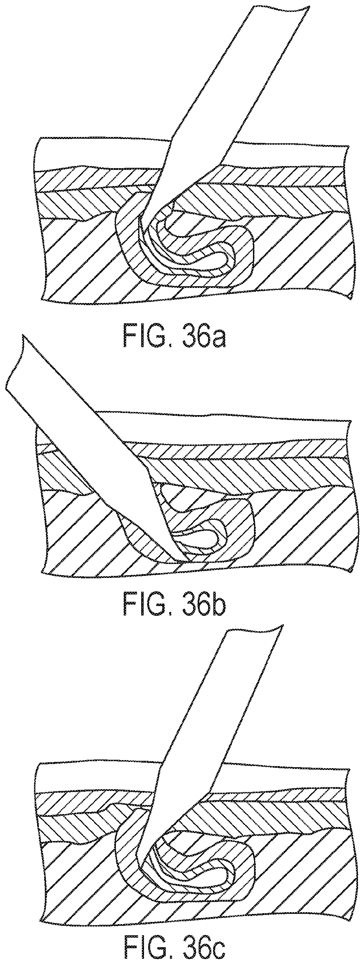

**FIG. 36 a-c** exemplarily illustrates the use of the frustoconical hair punch

### DETAILED DESCRIPTION OF CERTAIN EMBODIMENTS

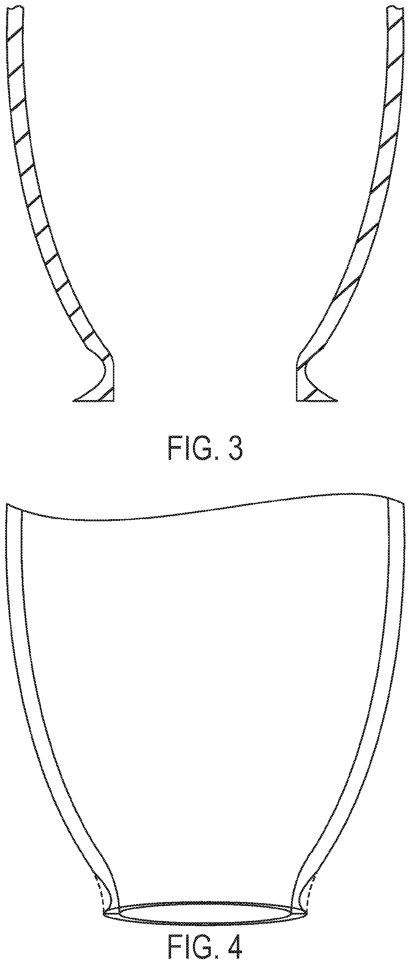

A punch 10 is employed to remove hair follicles. The punch 10 includes a generally tubular body 12 formed about a generally central axis 14 and extending from a generally tubular body proximal end 16 to a generally tubular body distal end 18. A generally frustoconical portion 20 further includes a generally frustoconical portion proximal end 22 and a generally frustoconical portion distal end 24. The generally frustoconical portion 20 is formed about the generally central axis 14. The generally frustoconical portion proximal end 22 is joined to the generally tubular body distal end 18. A generally tubular transition portion 26 has a generally tubular transition portion proximal end 28 and a generally tubular transition portion distal end 30. The generally tubular transition portion proximal end 28 is joined to the generally frustoconical portion proximal end 24. The generally tubular transition portion shown in Figure 1 ranges in length from 0 to 3mm. As shown in Figure 2, Figure 3 a cutting edge 32 is connected to the generally tubular transition portion. Inserting the cutting edge into skin creates a wound to remove the hair follicles. In some embodiments, the cutting edge can be serrated. In some embodiments, a cutting end 132, 232, 332, can be pronged for non-rotary use as shown in Figures 29A-C.

Turning to a cutting end 432 that is shown in more detail in Figures 30, 30A-C, the distal end region 434 of a rotating punch 410 constructed in accordance with the invention is provided with a textured exterior region 436. As the rotating punch 410 is pushed into the tissue, the textured region 436 appears to enhance the suction-like effect that gently sucks the cut tissue towards the punch as it descends into the tissue. It is believed that the texture creates mini vortices that impart a suction effect on the tissue surrounding a fast-rotating punch. In a rotating punch 410, the suction effect of these threaded textures results in a screw like effect that causes the punch to be self-forward-driven as well as exert an upward pulling effect on the tissue it contacts. The direct effect is that when the rotating punch 410 is laid at a very acute angle to the skin, and the cutting tip makes the first cut into the skin in the process, the external texture follows by engaging the skin through its threads and self-propels the punch towards the engulfed follicle. The pulling effect on tissue around the follicle results in a simultaneous cutting of follicular tissue attachments (anchoring system) by a preferably outward and away facing punch cutting tip. Currently, a preferred textured region is composed of thin lines of surface discontinuity approximately 12.5 micrometers deep and spaced approximately 0.15 mm apart. Those skilled in the art will recognize that the "lines" need not necessarily be straight or continuous and may comprise separated surface discontinuities following some pattern or arranged in a relatively random manner. Although the presently preferred textured region is comprised of lines that are concentric about the central axis 14 and extend 360° about the punch, the textured region may instead comprise lines that are helical and/or extend less than 360°. Further, the textured region may be continuous or may be formed in separate spaced-apart circular or helical segments so long as the desired effect is achieved. Moreover, the textured region can instead comprise randomly distributed discontinuities in the surface of the punch. The textured region preferably extends to the cutting edge but can be slightly spaced therefrom if one wishes to do so. External texturing can involve at least one section of the proximal five millimeters of the punch. The internal texturing can extend to the cutting tip or edge or be slightly spaced therefrom if one wishes to do so.

Punches constructed in accordance with the invention can also include an internal textured region 438 instead of or in addition to the textured external region described above. The interior textured region appears to help draw the follicle into the chamber; it is believed that, like the external texture region 436, the interior texture creates mini-vortices that impart a suction effect on the tissue follicle. Particularly pertinent to the internal texturing more than the external texturing is its direct upward pulling effect on the follicle engulfed by the punch causing the follicle to ascend into the proximal reaches of the punch lumen.

As shown in Figures 34A-C, the pulling effect on the follicle results in a simultaneous cutting of follicular tissue attachments (Anchoring systems) by a preferably outward and away facing punch cutting tip. In this scenario, hair follicles that take an angled path in the depths of the skin are relatively unharmed as the hair angle is pulled into the punch lumen ahead of the cutting tip of the punch as it is progressively plunged into the skin towards the root and bulb of the follicle. This property also improves the transection rate when the punch-follicle axes are relatively misaligned since the upward pull forces the graft in to the lumen ahead of the cutting tip similarly. Another effect of the internal texturing in a fast-rotating punch is the generation of a vortex or tornado effect 440 which creates a vortex whose influence includes a zone that is in front and of the punch cutting end, the vortex fans out from the cutting end opening to gather far flung follicles towards and into the narrower opening. The effect is that the punch opening is able to capture multi-haired follicles whose skin zone diameter exceeds the diameter of the punch. Preferably, the punch has both a textured external region 436 and an internal textured region 438.

The presently preferred internal textured region 438, preferably comprises surface discontinuities of the same size and spacing as the exterior textured region and is disposed over at least a part of the diverging interior portion of the punch as shown in Figure 30B. As with the exterior textured region, the interior textured region can comprise concentric lines that extend 360° about the punch, are helical and/or extend less than 360°. Further, the internal textured region can be continuous or formed in separate spaced-apart circular or helical segments or comprise randomly distributed discontinuities in the wall of the follicle-receiving chamber, so long as the desired effect is achieved. Currently, the preferable configuration is on which utilizes concentric interior and exterior lines that extend 360° about the punch. Preferably, the textured region extends to the cutting edge, but can be slightly spaced therefrom if one wishes to do so.

The generally tubular body 12 can further comprise a generally tubular body proximal end radius 34 measured from the generally central axis 14 to a generally tubular body inner wall 36 at the generally tubular body proximal end 16. A generally tubular body distal end radius 38 is measured from the generally central axis 14 to a generally tubular body inner wall 36 at the generally tubular body distal end 18. The generally tubular body proximal end radius 34 is approximately equal to the generally tubular body distal end radius 38.

The generally frustoconical portion 20 can further comprise a generally frustoconical portion proximal end radius 40 measured from the generally central axis 14 to a generally frustoconical portion inner wall 42 at the generally frustoconical portion proximal end 22. A generally frustoconical portion distal end radius 44 is measured from the generally central axis 14 to a generally frustoconical portion inner wall 42 at the generally frustoconical portion distal end 24. The generally frustoconical portion distal end radius 40 and the generally frustoconical portion proximal end radius 44 can vary in a number of proportions.

Additionally, there can be many combinations of the generally frustoconical portion distal end radius 44 and the generally frustoconical portion proximal end radius 40, though these dimensions are always different from one another. The cutting edge 32 further has a cutting edge radius 46 that is measured from the central axis 14 to the proximal end of the cutting edge. The cutting edge 32 can be the same as either of the generally frustoconical portion distal end radius 44 or the generally frustoconical portion proximal end radius 40. In many embodiments, the cutting edge radius is different. In some embodiments, the generally cylindrical body has a longitudinal axis. A slot can be cut through the punch along the longitudinal axis from the generally cylindrical portion to the cutting edge.

Turning to Figures 8-10, in some embodiments, the cutting end 532, 632, 732 includes a cutting tip 534, 634, 734 on the outer perimeter of the cutting end which 532, 632, 732 flared to point away from the tube's general central axis 14 in an angle that could ranges from 1-90 degrees, so that the cutting tip 534, 634, 734 can counteract the vectors of the cutting action of the expanding external wall 43 of the punch which tends to exert a cutting force directed toward central axis 14 and the follicle. To minimize the chance of follicle damage from this action, a flared cutting tip which directs the direction of the cutting force away from the central axis 14 (thus the follicle) is preferred. The flared cutting tip directs the cutting force away from the follicle to counteract the direction of the vectors created by the expanding cone as the punch advances into the skin, which is towards the follicle (and thus the central axis 14).

The flare of the cutting tip can be created by adding a concavity to the distal 1-3 mm of the external wall 43 of the tube. The concavity can be created by direct flaring of the tube as shown in Figure 2 or by etching it out of the external wall 43 of the tube to create a second frustoconical portion distal to the first frustoconical portion as shown in Figure 3. The flare can be curved (as shown in Figure 3) or it could be angled or stepped.

One problem reported by users of punches possessing a flared cutting tip that has a cutting tip directed away from the central axis 14 is that of buried grafts. This is particularly problematic in flared punches that have a cutting end inferior face whose plane is at approximately 90 degrees axis orientation to the central axis 14 especially if the plane is either flat as shown in Figure 8 or convex as shown in Figure 11 is that of a high incidence of buried grafts. Buried grafts are not available for use in transplantation and result in complications of inflammation, folliculitis, ingrown hair, cysts and abscesses as shown in Figure 32c. Buried grafts occur in these scenarios when the outer cutting tip incises the skin for an initial score, but the follicle and its circular skin to ascend into the lumen of the punch, it is pushed down into the skin by the blunt surface presented by the flat or convex surface. This is in part because the diameter of the cut skin defined by the very wide diameter of the cutting tip 536 far exceeds the diameter of the internal diameter of the punch cutting end 538 as shown in Figures 32A-C. One method for reducing this is to increase the very wide diameter of the cutting tip 536 by caving out of the internal wall of the distal end of the frustoconical a cylindrical swath that can reduce the tube wall thickness by as much as 50-75% as shown in Figure 14. The new opening diameter 540 would be significantly wider than the internal diameter of the punch cutting end 538 and the ratio of the new opening diameter 540 to the internal diameter of the punch cutting end 538 ameliorates the possibility of buried grafts significantly. In the setting of the frustoconical design, doing so would quickly expose the follicle cap to a rapidly expanded chamber which would significantly increase the ratio and minimize the incidence of buried grafts. Furthermore, the process of compressing a larger tube to a narrow frustoconical end, thickens the tube wall thickness at the junction between the frustoconical distal end and its connection to the cutting end. This gives more room to carve out and from the internal wall larger opening connecting the frustoconical chamber to the new opening diameter 540.

Another solution to buried grafts is to slant 561, 563 the cutting edge plane 32(as shown in Figure 8, 9, 15, and 33a-c) or texture it 552 to enable upward pulling of grafts (as shown in Figure 16, 13a, 13b, 33b and 33c). All of these individual measures could be used alone or in various combinations.

Finally, the expanded internal volume brought about by the frustoconicallity gives enough room to accommodate the large diameter of the skin scored 554 by the large tip diameter of the flared punch 536 (as shown in Figure 32a and 33c) preventing impaction in the absence of which torsion injury 558 and crushing of the distal portions 556 of the grafts could occur (as shown in Figure 26a, b and c). One factor that could potentially contribute to buried grafts is when the grafts is unable to ascend up the punch because of limited punch lumen capacity.

Turning to these dimensions in more detail. The generally tubular body proximal end radius 40 and the generally tubular body distal end radius 38 can range from .25 mm to 2.5 mm. The length of the transition portion along the central axis 14 can range from .5 mm to 5 mm. The inflection point of changing diameters can start at any point beginning from the cutting tip to anywhere along 5 mm from the cutting tip. Those practiced in the art recognize that these numbers may vary slightly outside these ranges. For instance, if the distal cutting tip is 0.5mm in diameter and the proximal end 28 diameter is 5mm, the ratio is 10:1. Although he preferred ration ranges from 3:1 to 1.12:1, the ration can be larger than this as well. Additionally, the ratio can be sustained or exceeded in a version whose diameter in normal plane with the cutting tip is 2.5mm.

The expanding internal cross-sectional area of the generally frustoconical portion 20 creates more room to accommodate the graft tissue gathering in the lumen, reduce friction between tissue and wall thereby reducing transection. Additionally, the expansion in chamber volume increases the suction force exerted on the graft which the fast rotating punch as well as the texturing has initiated. However, a fast rotating or oscillating punch without texturing may generate some suction energy without texturing due to Bernoulli like effect. Turning to the external surface, the expanding diameter exerts a blunt dissection effect on the tissue attachments of the follicle, thus enhancing the separation of the follicle from its attachment to the surrounding tissue. This minimizes the amount of secondary dissection the surgeon has to perform to extract the graft.

The distal end 30 of the punch has a cutting tip 534, 634, 734 which may be unsharpened or, preferably, sharpened. The cutting edge may be sharpened from the exterior of the punch 43 or, from its distal cutting edge surface 32 or from inner wall 42 of its lumen. There are many cutting edge shapes that can be used. The following are exemplary:

The cutting edge 32 is formed by the interior wall meeting the exterior wall at the distal cutting tip 534, 634, 734, but the internal wall 42 might not meet the external wall 43 but rather terminate a short distance away from it to create a blunt 560 or flat slant (561 or 563), convex 562 or concave 550 plane between the ends of the external wall 43 and the internal wall 42 ( as shown in Figure 8, 9, 11 and 12). While the distal end of the segment preferably terminates at the cutting edge where the external wall meets the internal wall, as illustrated in FIGS. 35 c and 35d, the internal wall may alternatively terminate just proximal of the cutting edge as depicted in FIGS 35 e and 35 f to create a blunt or flat or convex or concave plane in the gap between where the external and internal walls terminate without departing from the scope of the invention.

The external wall 43 surface of the distal end region of the punch may be further provided with generally circumferentially-extending notch having a generally concave shape 566 that generally circumscribes the punch's cutting edge 32 or the punch's outer surface 43 (as shown in Figure 2 and 6). The concavity preferably extends 1-2 mm proximally from a location closely adjacent the tip of the punch. The generally concave shape serves two purposes. First, its preferred size and shape results in a wound with less everted edges or shapes 572 (as shown in Figure 22c); as the punch enters the tissue surrounding the targeted follicle, the tissue outward of the cut expands 570 against the concavity as it is passed by the cutting tip 534 (as shown in Figure 22a). When the punch is subsequently withdrawn, the tissue resumes its consequently less everted shape 572. Second, the concavity on the outer wall 43 of the distal end 30 of the tube results in a sharp cutting tip 534 located on the perimeter of the cutting end whose cutting action is substantially directed away 574 from the central axis 14 of the tube and by consequence away from the follicle 576 engulfed by the cutting end. In so doing the risk of transection damage to the follicle is minimized as shown in Figure 22 a-c.

In some embodiments, the cutting end further comprises a pair of distally-extending, circumferentially disposed, prong-like members carrying distally diverging cutting edges 132, 232, 332 separated by follicle-accommodating slits 250, 252, 254 as shown in Figure 29 A-E. The prong-like members are arranged to terminate distally in respective leading cutting tips that make the initial penetration into tissue surrounding the follicle, and a distal end region 434 of the follicle punch includes a circumferentially-extending concave groove that circumscribes an outer surface of the follicle punch. The circumferentially-extending notch can extend distally 1-2 mm from tips of the prong-like members.

Alternately, the cutting end can further comprise a pair of distally-extending, circumferentially disposed, prong-like members carrying distally diverging cutting edges 132, 232, 332 separated by follicle-accommodating slits 250, 252, 254 wherein the follicle-accommodating slits 250, 252, 254 each have an inverted V profile as shown in Figure 29 A, D and E. The inverted V profile can comprise a relatively proximal segment and a distal segment that is more steeply tapered than the distal cutting end region 434. The prongs can be flared to direct the cutting edges 132, 232, 332 away from the central axis 14 by an angle between 1 and 60 degrees as shown in Figure 29D-E. Alternately, the prongs can be flared to direct the cutting tips 534, 634, and 734 (as shown in Figure 8-10) away from the central axis 14 by an angle of between 1 and 60 degrees.

In some embodiments, one or more of the cutting edges 132, 232, 332 can be blunt. Alternately, one or more of the cutting edges 132, 232, 332 can be sharp. Additionally, the two slits can come to an end distally at differential levels that range from 0.25 to 1.5mm as shown in Figures 29 B and C.

Alternatively, the punch can be provided with a flared distal end region 434 having a distal end radius 44 diameter that has a diverging inner diameter and diverging outer diameter along the distal end 30. The distal portion can be at least 0.1 mm but no more than 5.0 mmm and preferably about 1.0 mm with the flared end region resulting in a preferred gap of approximately 1.25 mm between opposing tips. Gaps of great or lesser spacing may be utilized as well, depending on the subject's hair and follicle dimensions without departing from the scope of the invention.

In one configuration, the cutting edge 232 can have a general inverted "V" profile 234 having a relatively proximal segment 236 and a relatively distal segment 238 that is more steeply tapered than the proximal segment as shown in Figure 29. The more steeply tapered interior of the relatively distal segment provides a slit length and width that accommodates the follicle as the punch penetrates the surrounding tissue, in order to spare the follicle from being cut. The less tapered proximal segment of the slit results in adequate spacing of the cutting edges of adjacent prong-like members from the follicle's root structure so that the cutting yields a viable implant. Although the same taper could be used for both segments, it is preferable not to do so since a generally uniformly steep taper, such as that of the preferred distal segment, would add unnecessary length to the punch to achieve the needed spacing between the prongs, while a generally uniformly shallow taper, such as that of the preferred proximal segment, would fail to provide the slit length needed.

As show in Figures 30 and 30 A-C, the cutting edge 32 of the punch, which preferably extends from its leading tip to the beginning of the steeply tapered portion of the slit (i.e., the interface of the proximal and distal slit segments), may be smooth 438 or textured 436 to include one or more serrations on the internal 42 or external walls 43. If serrations are included, it is currently preferable that there be one or two serrations, with rounded edges, although the use of sharply angled edges would not depart from the scope of the invention.

The external diameter expanding with depth has many benefits such as the skin wound is small diameter 578 and the remaining graft separating dissection occurs by blunt dissection (as shown in Figure 27, 27a, 27b, 31a and 27c ). This results in a lesser need for sharp dissection which carries a lesser chance of cutting into the follicles and transecting them. Next, the expanding diameter 580 aids in the separation of tissue attachments as the punch progresses in depth. This results in less need for secondary dissection needed to remove the grafts after the punch score. Additionally, because there is increased friction as the punch is advanced into the skin on account of the expanding diameter 580, this serves as a depth control mechanism (as shown in Figure 31b). The control mechanism can be communicatively coupled to the motor driven driveshaft and programmed with instructions to retard or stop driveshaft speed of the motor-driven driveshaft as the punch penetrates the skin deeper to though the frustoconical segment. The expanding diameter 580 begins from the point where the second frustoconical portion ends. In some embodiments, there is not a second frustoconical portion, rather the cutting portion begins from the point where the first general frustoconical portion terminates 24.

Furthermore, increasing diameter caused by the frustoconicality would cause an increase in friction upon the rotating or oscillating punch as it is progressively advanced into the skin resulting in slowing down or even stopping of the rotation. This serves two purposes: Firstly, it diminishes the cutting action of the cutting tip 534 and retards it further progression into the tissue which serves as depth control mechanism (as shown in Figure 31b). Secondly, it results in a less cutting action directed at the follicles which reduces the chance of cutting into the follicle (as shown in Figure 31b). Finally in lower torque situations the rotary motion self-converts to a brief oscillatory motion as the motor attempts to gather momentum and resume its rotation. The resulting cutting action and the toggle aids in further separating the distal portion of the follicle from its attachments to adjacent tissues

Additionally, the internal diameter expanding 582 with depth provides the following benefits. The internal diameter expanding 582 with progressing depth gives the room for the engulfed graft to expand with less friction between generally tubular body inner wall 36 or general frustoconical portion inner wall 42 and graft resulting in less heat generation and less heat damage to graft (as shown in Figure 28A-B). The internal diameter expanding 582 with progressing depth gives the room for the engulfed graft to expand with less friction between generally tubular body inner wall 36 or general frustoconical portion inner wall 42 and graft (as shown in Figure 28A-B). Without this action, the graft sticks the rotating tube 558 which results in torsion of the graft eventuating in decapitation or transection of the graft 584 (as shown in Figure 26A). That is, graft damage. The internal diameter expanding 582 with progressing depth giving room for the engulfed graft to expand with less friction between tube and graft. Without this action, the graft sticks the tube. In order for the punch to go all the way to the bottom of the graft that it engulfs, the graft needs to migrate up the punch lumen to make room. In the absences of this, the punch and graft descend on the bottom portions of the graft as one block, crushing it 556 in the process (as shown in Figure 26B-C). The internal diameter expanding 582 with progressing depth giving room for the engulfed graft to expand 582 (shown in figure 28b). This enables bulkier grafts 586 to be retrieved. Bulkier grafts 586 (i.e. grafts with more tissue invested around them) survive better once removed (as shown in Figure 33B-C).

In some cases, as shown in Figure 30 and Figures 30A-C, the device can be altered with fine adjustments. As there would be increasing resistance as the tool is advanced into the skin because of the expanding size, measures can be taken to reduce the resistance and enhance tissue penetration. Such measures can include but not limited to, first, creation of internal textures 438 (grooves that are in helical, partially or completely circular) that give a screw like effect in a rotating punch. Next, creation of external textures 436 (grooves that are in helical, partially or completely circular) that give a screw like effect in a rotating punch. Longitudinal grooves that aid or guide penetration when a punch is used in a plunging manner rather than a rotary manner. Longitudinal grooves could be of any length or depth. Finally, horizontal grooves that are complete or partially disposed in the frustoconical area that exert a ribbed like effect to enhance forward advancement when punch is advanced in a plunging manner. Any of these grooves or textures could be located in a complete or partial parts of the frustoconical area. Additionally, these grooves, could be located in front of the frustoconical area to have a pulling effect on it to aid its ability to penetrate the tissue despite its increasing width. A suction device could be either in front of, within or behind the frustoconical area.

The suction effect could be increased by increasing an internal diameter of the distal end radius 44 of the wall to achieve a Bernoulli effect either in front, within or behind the frustoconical area. mechanical movement of the punch to drive the into the tissue that could be adjusted by a driver that could be rotary, oscillatory, vibratory, ultrasonic, pneumatic etc. Friction between tissue and frustoconical areas could be adjusted by polishing, lubrication, manipulation of electrostatic properties, and temperature control of the generally tubular member. Patterned or unpatterned wall openings of various sizes and shapes could be arranged within the frustoconical area.

[B]. The frustoconical shape inherently results in a blunt dissection effect that results in the separation 588 of tissue such as hair follicle within lumen of tube from tissue outside the tube with minimal stress on the tissue within the lumen as would occur in a method that solely relies on the cutting action of the cutting tip 534 that lies ahead of the frustoconical portion (as shown in Figure 31a, 31b). This blunt dissection effect can be enhanced by additional features that include but are not limited to:
[A]. Texturing of various configurations and grit sizes / coarseness
[B]. Cobblestonning of various sizes and configurations
[C]. Patterned or unpatterned projections or indentations such as grooving, hexing, ridging, serrating.
[D]. Patterned or unpatterned wall openings of various sizes and shapes within the frustoconical portion area
[E]. For the frustoconical tube to penetrate the skin / tissue, it would need a cutting tip which could be in distal to or within the frustoconical portion section. It is preferably at the end of the frustoconical portion or within 0-3 millimeters of the end of the frustoconical portion.
[F]. The cutting tip could assume any configuration such as curved, stepped, angled, round, smooth, serrated, completely or partially circular, oval, rectangular, triangular, hexagonal, 2 pronged, 3- pronged, 4- pronged). A cross section of the frustoconical tube can be round, completely or partially circular, oval, rectangular, triangular, hexagonal, or any similar shape. While a substantially smooth concave segment is preferred, the segment can alternatively be stepped 568 or angled 566 without departing from the scope of the invention, and these variations are intended to be included within the term "concave" as used herein. (as shown in FIG. 6 and 7)
[G]. The cutting edge 32 could be advanced in a rotary, oscillatory or simply pushed into the tissue without any rotary or oscillatory motion.
[H]. The external wall 43 of the cutting end relative to the punch's general central axis 14 on a lateral profile can be straight, curved without any rotary or oscillatory motion.
[I]. The external wall 43 of the cutting end relative to the punch's general central axis 14 on a lateral profile (between the cutting tip and the frustoconical portion or within the frustoconical portion) can be concave, convex, straight, curved.
[J]. The external wall 43 of the cutting edge 32 relative to the punch's general central axis 14 on a lateral profile (between the cutting tip and the frustoconical portion 20or within the frustoconical portion 20) can have patterned or unpatterned wall openings of various sizes and shapes within the frustoconical portion area 20.
[K]. The cutting edge 32 relative to the punch's general central axis 14 on a lateral profile that is viewed from the punch central axis 14 to the cutting tips 534, 634, 734 could terminate in a flat plane at 90 degrees 560 (as shown in FIG. 8) or be slanted 561 in an angle that ranges from 85 degrees to 20 degrees in a manner that increases the sharpness of the cutting end with decreasing angulation FIG 9.
[L]. The cutting edge 32 relative to the punch's general central axis 14 on a lateral profile that is viewed from the punch center axis 14 to the cutting tip could terminate in an elevated flat plane 563 at between 95 degrees and 145 degrees FIG 10.
[M]. The cutting edge plane 32 relative to the punch's general central axis 14 on a lateral profile can terminate in an outwardly pointing sharp cutting edge 563.( as shown in FIG. 10)

The cutting edge surface 32 viewed from its distal inferior face viewed from the punch's center axis 14 to the outer margins where the cutting tip is located, can be concave 550 or convex 562. (as shown in Figure 11 & 12 respectively).

The cutting end surface 32 viewed from its distal inferior face viewed from the punch's center axis 14 to the outer margins where the cutting tip is located, can be concave 550 or convex 562 in a plane that is at 90 degrees measured between the central axis 14 and the cutting tip. (as shown in Figure 11 & 12 respectively).

The cutting edge surface 32 viewed from its distal inferior face viewed from the punch's center axis 14 to the outer margins where the cutting tip is located, can be concave 550 or convex 562 in a inwardly pointing plane that is at between 85 and 20 degrees 561 . (as shown in Figure 11 , 12 and 9 respectively)

The cutting edge surface 32 viewed from its distal inferior face viewed from the punch's center axis 14 to the outer margins where the cutting tip is located, can be concave 550 or convex 562 in an outwardly pointing plane that is at between 95 and 145 degrees 563. Figure 11 , 12 and 10 respectively)

The interface between where the largely vertical of proximally diverging internal wall the frustoconical chamber 42 and where it begins to diverge distally towards the laterally located cutting edge 32 located on the external wall 43 can be a smooth convex 564(as shown in Figure 5), or smooth concave 566 (as shown in Figure 6) or a smooth ridged wall or alternatively assume a stepped profile 568(as shown in Figure 7).

The cutting edge surface 32 viewed from its distal inferior face viewed from the punch's center axis 14 to the outer margins 532, 632, 732 where the cutting tips 534, 634, 734 located can be smooth (as shown in Figure 8-12), or textured. (as shown in Figure 13A-B) If textured, the preferred texture would be a continuation of the internal texturing described above, this is to enjoy the graft pulling vortex effect 440 already described in these sections.

The frustoconical punch can be couples to a hand piece through which liquid fluid is channeled from its proximal end through its lumen to egress at its cutting edge 32. This brings several advantages. First; The fluid serves to lubricate the internal wall lining 590 of the punch which reduces impaction and the problem of torsion transection (as shown in Figure 26a) and crushing injury 556 to distal follicle parts (as shown in Figure 26 b & c). Secondly; The fluid forms a hydroplane on the skin around the follicle which reduces the drag on the punch tip as it cuts into the skin. This reduces punch tip wear rate as well as forms an airtight seal between the punch and skin to enhance the advantages of graft suction effect of internal texturing as well as Bernoulli effect from frustoconical volume expansion 582 of the punch lumen. (as shown in Fig. 28b) Thirdly, the fluid is deposited in the space 588 created around the follicles when the punch scores around it. (as shown in FIG. 31b) The fluid envelops the freed graft and serves to sustain it in the time between the score and the transfer of the graft to a storage solution. The graft is otherwise susceptible to the deleterious effects of desiccation in that time interval. Finally; by manipulating pressure of fluid egress, grafts and debris cut in the punch lumen can be ejected by pulse fluid ejection. Conventionally, the operator must stop procedure and manually remove graft using forceps (as shown in Fig. 27b) or worse, sacrifice it by pushing it further into the proximal ends 28 of the punch lumen.

As used in this application, the term "a" or "an" means "at least one" or "one or more."

As used in this application, the term "about" or "approximately" refers to a range of values within plus or minus 10% of the specified number.

As used in this application, the term "substantially" or "generally" means that the actual value is within about 10% of the actual desired value, particularly within about 5% of the actual desired value and especially within about 1% of the actual desired value of any variable, element or limit set forth herein.

Persons of ordinary skill in the art may appreciate that numerous design configurations may be possible to enjoy the functional benefits of the inventive systems. Thus, given the wide variety of configurations and arrangements of embodiments of the present invention the scope of the invention is reflected by the breadth of the claims below rather than narrowed by the embodiments described above.

## Claims

1. A punch (10), for removing hair follicles; the punch comprising:
a generally tubular body (12) formed about a generally central axis (14) and extending from a generally tubular body proximal end (16) to a generally tubular body distal end (18);
a generally frustoconical portion (20), terminating in a generally frustoconical portion distal cutting end (24);
wherein, an outer perimeter of the generally frustoconical portion distal cutting end is defined by a sharp cutting tip (32) which extends and points away from the central axis in angles ranging from 30 degrees to 120 degrees to direct the cutting forces substantially away from hair follicle;
wherein the cutting end creates a wound to remove hair follicles;
wherein the generally frustoconical portion further comprises a generally frustoconical proximal end (22) having a frustoconical proximal end radius (40) from the generally central axis;
wherein the generally frustoconical portion distal cutting end has a frustoconical distal end radius (46) from the generally central axis; and
wherein a frustoconical axis ratio between the frustoconical distal end radius and the frustoconical proximal end radius is between about 1:3 and about 1:1.10.

2. The punch of claim 1 wherein, an external wall of the generally frustoconical portion distal cutting end relative to the punch's general central axis on a lateral profile is straight.

3. The punch of claim 1 wherein, an external wall of the generally frustoconical portion distal cutting end relative to the punch's general central axis on a lateral profile is curved in a concave manner to terminate in a cutting tip that extends and points outward.

4. The punch of claim 3 wherein the generally frustoconical portion distal cutting end continues in circular concave course that terminates at the cutting tip, the cutting tip pointing outwards and downwards relative to the central axis.

5. The punch of claim 3 wherein the generally frustoconical portion distal cutting end continues in circular concave course that terminates at the cutting tip, the cutting tip pointing outwards and at 90 degrees relative to the central axis.

6. The punch of claim 3 wherein the generally frustoconical portion distal cutting end continues in circular concave course that terminates at the cutting tip, the cutting tip pointing outwards and proximally at between 95-120 degrees relative to the central axis.

7. The punch of claim 3 wherein the cutting end relative to the punch's general central axis on a lateral profile that is viewed from the punch center to the cutting tip that terminates in a flat plane at 90 degrees.

8. The punch of claim 3 wherein the cutting end relative to the punch's general central axis on a lateral profile that is viewed from the punch center to the cutting tip that is slanted in an angle that ranges from 85 degrees to 20 degrees in a manner that increases the sharpness of the cutting end with decreasing angulation.

9. The punch of claim 3 wherein the cutting end surface viewed from its distal inferior face viewed from the punch's center to the outer margins where the cutting tip is located is textured.

10. The punch of claim 9 wherein the texturing includes lines of discontinuities approximately 12.5 micrometers deep.

11. The punch of claim 9 wherein the texturing includes lines of discontinuities are generally concentric about the generally central axis.

12. The punch of claim 9 wherein the texturing includes generally lines of discontinuities that are approximately 0.15 mm apart.

13. The punch of claim 3, wherein a distal end region of the punch has an exterior textured region that does not extend through a thickness of the generally cylindrical body.

14. The punch of any one of the preceding claims, wherein the generally frustoconical portion distal cutting end comprises a straight walled cylindrical transition zone (26) of variable length ranging from 0-3mm.

15. The punch of any one of the preceding claims, wherein the generally frustoconical portion further comprises an internal diameter that expands with depth and an external diameter that expands with depth.

## Patentansprüche

1. Stanze (10) zum Entfernen von Haarfollikeln; wobei die Stanze aufweist:
einen allgemein rohrförmigen Körper (12), der um eine allgemein zentrale Achse (14) gebildet ist und von einem proximalen Ende (16) des allgemein rohrförmigen Körpers zu einem distalen Ende (18) des allgemein rohrförmigen Körpers verläuft;
einen allgemein kegelstumpfförmigen Abschnitt (20), der in einem distalen Schneidende (24) des allgemein kegelstumpfförmigen Abschnitts endet;
wobei ein Außenumfang des distalen Schneidendes des allgemein kegelstumpfförmigen Abschnitts durch eine scharfe Schneidspitze (32) definiert ist, die unter Winkeln im Bereich von 30 Grad bis 120 Grad von der zentralen Achse verläuft und weg weist, um die Schneidkräfte im Wesentlichen von dem Haarfollikel weg zu lenken;
wobei das Schneidende eine Wunde zum Entfernen von Haarfollikeln erzeugt;
wobei der allgemein kegelstumpfförmige Abschnitt ferner ein allgemein kegelstumpfförmiges proximales Ende (22) mit einem Radius (40) des kegelstumpfförmigen proximalen Endes von der allgemein zentralen Achse aufweist;
wobei das distale Schneidende des allgemein kegelstumpfförmigen Abschnitts einen Radius (46) des kegelstumpfförmigen distalen Endes von der allgemein zentralen Achse aufweist; und
wobei ein kegelstumpfförmiges Achsenverhältnis zwischen dem Radius des kegelstumpfförmigen distalen Endes und dem Radius des kegelstumpfförmigen proximalen Endes zwischen etwa 1:3 und etwa 1:1,10 ist.

2. Stanze nach Anspruch 1, wobei eine Außenwand des distalen Schneidendes des allgemein kegelstumpfförmigen Abschnitts relativ zu der allgemein zentralen Achse der Stanze in einem seitlichen Profil gerade ist.

3. Stanze nach Anspruch 1, wobei eine Außenwand des distalen Schneidendes des allgemeinen kegelstumpfförmigen Abschnitts relativ zu der allgemein zentralen Achse der Stanze in einem seitlichen Profil in einer konkaven Weise gekrümmt ist, um in einer Schneidspitze, die nach außen verläuft und weist, zu enden.

4. Stanze nach Anspruch 3, wobei sich das distale Schneidende des allgemein kegelstumpfförmigen Abschnitts in einem kreisförmigen konkaven Verlauf, der bei der Schneidspitze endet, fortsetzt, wobei die Schneidspitze nach außen und nach unten relativ zu der zentralen Achse weist.

5. Stanze nach Anspruch 3, wobei sich das distale Schneidende des allgemein kegelstumpfförmigen Abschnitts in einem kreisförmigen konkaven Verlauf, der bei der Schneidspitze endet, fortsetzt, wobei die Schneidspitze nach außen und im 90-Grad-Winkel relativ zu der zentralen Achse weist.

6. Stanze nach Anspruch 3, wobei das distale Schneidende des allgemein kegelstumpfförmigen Abschnitts in einem kreisförmigen konkaven Verlauf, der bei der Schneidspitze endet, fortsetzt, wobei die Schneidspitze nach außen und proximal im Winkel zwischen 95-120 Grad relativ zu der zentralen Achse weist.

7. Stanze nach Anspruch 3, wobei das Schneidende relativ zu der allgemein zentralen Achse der Stanze in einem seitlichen Profil, das von der Stanzenmitte zu der Schneidspitze gesehen ist, in einer flachen Ebene bei 90 Grad endet.

8. Stanze nach Anspruch 3, wobei das Schneidende relativ zu der allgemein zentralen Achse der Stanze in einem seitlichen Profil, das von der Stanzenmitte zu der Schneidspitze gesehen ist, unter einem Winkel im Bereich von 85 Grad bis 20 Grad in einer Weise geneigt ist, dass die Schärfe des Schneidendes mit abnehmender Winkelbildung zunimmt.

9. Stanze nach Anspruch 3, wobei die Schneidenden-Oberfläche, von ihrer distalen unteren Fläche gesehen, von der Mitte der Stanze zu den Außenrändern, wo sich die Schneidspitze befindet, gesehen, texturiert ist.

10. Stanze nach Anspruch 9, wobei die Texturierung näherungsweise 12,5 Mikrometer tiefe Sprunglinien enthält.

11. Stanze nach Anspruch 9, wobei die Texturierung Sprunglinien enthält, die um die allgemein zentrale Achse allgemein konzentrisch sind.

12. Stanze nach Anspruch 9, wobei die Texturierung allgemein Sprunglinien, die näherungsweise 0,15 mm auseinander sind, enthält.

13. Stanze nach Anspruch 3, wobei ein distales Endgebiet der Stanze ein texturiertes Außengebiet aufweist, das nicht durch eine Dicke des allgemein zylindrischen Körpers verläuft.

14. Stanze nach einem der vorhergehenden Ansprüche, wobei das distale Schneidende des allgemein kegelstumpfförmigen Abschnitts eine geradwandige zylindrische Übergangszone (26) mit variabler Länge in dem Bereich von 0 3 mm aufweist.

15. Stanze nach einem der vorhergehenden Ansprüche, wobei der allgemein kegelstumpfförmige Abschnitt ferner einen Innendurchmesser, der sich mit der Tiefe erweitert, und einen Außendurchmesser, der sich mit der Tiefe erweitert, aufweist.

## Revendications

1. Un poinçon (10) permettant de retirer des follicules pileux ; le poinçon comprenant :
un corps généralement tubulaire (12) formé autour d'un axe généralement central (14) et s'étendant d'une extrémité proximale (16) du corps généralement tubulaire à une extrémité distale (18) du corps généralement tubulaire ;
une partie généralement tronconique (20), se terminant par une extrémité de coupe distale (24) de la partie généralement tronconique;
dans lequel un périmètre extérieur de l'extrémité de coupe distale de la partie généralement tronconique est défini par une pointe de coupe acérée (32) qui s'étend et s'éloigne de l'axe central dans un angle allant de 30 à 120 degrés afin d'éloigner les forces de coupe considérablement du follicule pileux ;
dans lequel l'extrémité de coupe crée une plaie pour retirer des follicules pileux ;
dans lequel la partie généralement tronconique comprend en outre une extrémité proximale généralement tronconique (22), présentant un rayon d'extrémité proximale tronconique (40) par rapport à l'axe généralement central ;
dans lequel l'extrémité de coupe distale de la partie généralement tronconique présente un rayon d'extrémité distale tronconique (46) par rapport à l'axe généralement central ; et
dans lequel un rapport d'axe tronconique entre le rayon de l'extrémité distale tronconique et le rayon de l'extrémité proximale tronconique est compris entre environ 1:3 et environ 1:1,10.

2. Le poinçon selon la revendication 1, dans lequel une paroi externe de l'extrémité de coupe distale de la partie généralement tronconique par rapport à l'axe généralement central du poinçon sur un profil latéral est droite.

3. Le poinçon selon la revendication 1, dans lequel une paroi externe de l'extrémité de coupe distale de la partie généralement tronconique par rapport à l'axe généralement central du poinçon sur un profil latéral est incurvée de manière concave pour se terminer par un point de coupe qui s'étend et pointe vers l'extérieur.

4. Le poinçon selon la revendication 3, dans lequel l'extrémité de coupe distale de la partie généralement tronconique se poursuit par une trajectoire circulaire concave qui se termine par la pointe de coupe, cette dernière étant orientée vers l'extérieur et vers le bas par rapport à l'axe central.

5. Le poinçon selon la revendication 3, dans lequel l'extrémité de coupe distale de la partie généralement tronconique se poursuit par une trajectoire circulaire concave qui se termine par la pointe de coupe, cette dernière étant orientée vers l'extérieur et à 90 degrés par rapport à l'axe central.

6. Le poinçon selon la revendication 3, dans lequel l'extrémité de coupe distale de la partie généralement tronconique se poursuit par une trajectoire circulaire concave qui se termine par la pointe de coupe, cette dernière étant orientée vers l'extérieur et proximalement entre 95 et 120 degrés par rapport à l'axe central.

7. Le poinçon selon la revendication 3, dans lequel l'extrémité de coupe est située par rapport à l'axe généralement central du poinçon sur un profil latéral, vu du centre du poinçon vers la pointe de coupe qui se termine par un plan plat à 90 degrés.

8. Le poinçon selon la revendication 3, dans lequel l'extrémité de coupe est située par rapport à l'axe généralement central du poinçon sur un profil latéral, vu du centre du poinçon vers la pointe de coupe qui est inclinée selon un angle allant de 85 degrés à 20 degrés, de manière à augmenter le tranchant de l'extrémité de coupe avec une angulation décroissante.

9. Le poinçon selon la revendication 3, dans lequel la surface de l'extrémité de coupe, vue de sa face inférieure distale depuis le centre du poinçon jusqu'aux bords extérieurs où se trouve la pointe de coupe, est texturée.

10. Le poinçon selon la revendication 9, dans lequel la texturation comprend des lignes de discontinuité d'environ 12,5 micromètres de profondeur.

11. Le poinçon selon la revendication 9, dans lequel la texturation comprend des lignes de discontinuité qui sont généralement concentriques autour de l'axe généralement central.

12. Le poinçon selon la revendication 9, dans lequel la texturation comprend généralement des lignes de discontinuité qui sont distantes d'environ 0,15 mm.

13. Le poinçon selon la revendication 3, dans lequel une région de l'extrémité distale du poinçon présente une région texturée extérieure qui ne s'étend pas à travers l'épaisseur du corps généralement cylindrique.

14. Le poinçon selon l'une des revendications précédentes, dans lequel l'extrémité de coupe distale de la partie généralement tronconique comprend une zone de transition cylindrique à paroi droite (26) d'une longueur variable allant de 0 à 3 mm.

15. Le poinçon selon l'une des revendications précédentes, dans lequel la partie généralement tronconique comprend en outre un diamètre interne qui s'élargit avec la profondeur et un diamètre externe qui s'élargit avec la profondeur.
